(19) Europäisches Patentamt
European Patent Office
Office européen des brevets

(11) Veröffentlichungsnummer: **0 125 409**
A2

# (12) EUROPÄISCHE PATENTANMELDUNG

(21) Anmeldenummer: 84102373.2

(22) Anmeldetag: 06.03.84

(51) Int. Cl.³: C 07 D 249/08
C 07 D 233/60, A 01 N 43/64
A 01 N 43/50

(30) Priorität: 15.04.83 DE 3313941

(43) Veröffentlichungstag der Anmeldung:
21.11.84 Patentblatt 84/47

(84) Benannte Vertragsstaaten:
BE CH DE FR GB IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Bühmann, Ulrich, Dr.
Fechnerstrasse 5
D-1000 Berlin 31(DE)

(72) Erfinder: Krähmer, Hansjörg, Dr.
Fürstendamm 10a
D-1000 Berlin 28(DE)

(72) Erfinder: Arndt, Friedrich, Dr.
Mühlenfeldstrasse 10
D-1000 Berlin 28(DE)

(72) Erfinder: Baumert, Dietrich, Dr.
Schulzendorfer Strasse 108b
D-1000 Berlin 28(DE)

## (54) 4-Azolyl-Pentannitrile, Verfahren zur Herstellung dieser Verbindungen sowie diese enthaltende biozide Mittel.

(57) Die Erfindung betrifft neue 4-Azolyl-pentannitrile der allgemeinen Formel

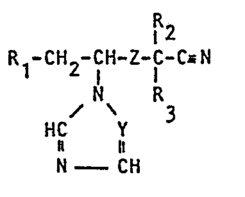

I

in der

$R_1$ einen gegebenenfalls ein- oder mehrfach, gleich oder verschieden durch Halogen, ($C_1$-$C_4$)-Alkyl, ($C_1$-$C_4$)-Alkoxy, ($C_1$-$C_4$)-Alkylthio, Trifluormethyl, Cyan und/oder die Nitrogruppe substituierten aromatischen Kohlenwasserstoffrest,

Y ein N-Atom oder die CH-Gruppe,

Z die Carbonyl- oder die CH(OH)-Gruppe und

$R_2$ und $R_3$ gleich oder verschieden sind und jeweils einen gegebenenfalls durch Halogen oder ($C_1$-$C_4$)-Alkoxy substituierten ($C_1$-$C_6$)-Alkylrest, einen ($C_3$-$C_6$)-Cycloalkylrest, einen ($C_3$-$C_6$)-Alkenyl- oder einen ($C_3$-$C_6$)-Alkinylrest, einen gegebenenfalls durch Halogen substituierten Phenyl-($C_1$-$C_4$)-alkylrest oder gemeinsam einen Alkylenrest

$$-(CH_2)_n-$$

bedeuten, worin n die Zahlen 2, 3, 4, 5 und 6 darstellt und deren Säureadditionssalze mit anorganischen und organischen Säuren.

Verfahren zur Herstellung dieser Verbindungen sowie diese enthaltende biozide Mittel.

EP 0 125 409 A2

Die Erfindung betrifft neue 4-Azolyl-Pentannitrile, Verfahren zur Herstellung dieser Verbindungen sowie diese enthaltende biozide Mittel insbesondere mit fungizider und pflanzenwachstumsregulatorischer Wirkung.

4-Azolyl-Derivate mit biozider Wirkung sind bereits bekannt (DE-OS-2 734 442; DE-OS 2 737 489; DE-OS 2 734 426; DE-OS 2 805 227; DE-OS 3 048 266; DE-OS 3 048 267).

Aufgabe der vorliegenden Erfindung ist die Zurverfügungstellung neuer 4-Azolyl-Derivate mit überlegenen Eigenschaften.

Diese Aufgabe wird erfindungsgemäß gelöst durch neue 4-Azolyl-pentannitrile der allgemeinen Formel

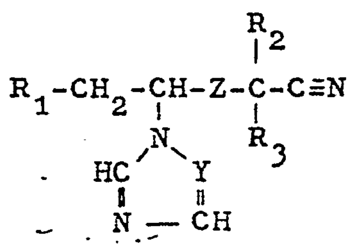

I

in der

$R_1$ einen gegebenenfalls ein- oder mehrfach, gleich oder verschieden durch Halogen, $(C_1-C_4)$-Alkyl, $(C_1-C_4)$-Alkoxy, $(C_1-C_4)$-Alkylthio, Trifluormethyl, Cyan und/oder die Nitrogruppe substituierten aromatischen Kohlenwasserstoffrest,

Y ein N-Atom oder die CH-Gruppe,

Z die Carbonyl- oder die CH(OH)-Gruppe und

$R_2$ und $R_3$ gleich oder verschieden sind und jeweils einen gegebenenfalls durch Halogen oder $(C_1-C_4)$-Alkoxy substituierten $(C_1-C_6)$-Alkylrest, einen $(C_3-C_6)$-Cycloalkylrest, einen $(C_3-C_6)$-Alkenyl- oder einen $(C_3-C_6)$-Alkinylrest, einen gegebenenfalls durch Halogen substituierten Phenyl-$(C_1-C_4)$-alkylrest oder gemeinsam einen Alkylenrest

$$-(CH_2)_n-$$

-2-

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG, Berlin, Konto-Nr. 8870600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 415008, Bankleitzahl 100 700 00 Postscheckamt Berlin West, Konto-Nr. 1175-101, Bankleitzahl 100 100 10

bedeuten, worin n die Zahlen 2, 3, 4, 5 und 6 darstellt und deren Säureadditionssalze mit anorganischen und organischen Säuren.

Die erfindungsgemäßen Verbindungen sind im weitesten Sinne biozid wirksam, zeichnen sich jedoch insbesondere durch eine fungizide und wuchsregulatorische Wirkung aus, worin sie überraschenderweise bekannte Wirkstoffe analoger Konstitution und Wirkungsrichtung übertreffen.

Die fungizide Wirkung erstreckt sich überraschenderweise gegen Pilze unterschiedlichster systematischer Stellung. Bei der Behandlung oberirdischer Pflanzenteile schützen sie gegen windbürtige Krankheitserreger. Gegen Samenbürtige Krankheitserreger kann man die Verbindungen zur Saatgutbehandlung einsetzen. Außerdem wirken diese systemisch, das heißt, sie werden von den Wurzeln der Pflanzen zum Beispiel nach der Saat aufgenommen, werden in die oberirdischen Teile der Pflanzen transportiert und schützen diese gegen Krankheitserreger.

Die erfindungsgemäßen Verbindungen weisen auch eine bakterizide Wirkung auf.

Wegen des erkannten breiten Wikrungsspektrums eignen sich die Verbindungen nicht nur zum Schutz von Kulturpflanzen sondern auch zum Materialschutz und zur Bekämpfung humanpathogener und tierpathogener Mikroben, woraus sich breit gefächerte Anwendungsmöglichkeiten ergeben.

Da die erfindungsgemäßen Verbindungen außerdem sowohl qualitative und quantitative Veränderungen der Pflanzen verursachen, sind sie in die Klasse der Pflanzenwachstumsregulatoren einzustufen, die sich durch folgende Anwendungsmöglichkeiten auszeichnen:



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Brunn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG, Berlin, Konto-Nr. 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

Hemmung des vegetativen Wachstums bei holzigen und krautigen Pflanzen zum Beispiel an Straßenrändern, Gleisanlagen u.a., um ein zu üppiges Wachstum zu unterbinden. Wuchshemmung beim Getreide, um das Lagern oder Umknicken zu unterbinden, bei Baumwolle zur Ertragserhöhung.

Beeinflussung der Verzweigung von vegetativen und generativen Organen bei Zier- und Kulturpflanzen zur Vermehrung des Blütenansatzes oder bei Tabak und Tomate zur Hemmung von Seitentrieben.

Verbesserung der Fruchtqualität, zum Beispiel eine Zuckergehaltssteigerung beim Zuckerrohr, bei Zuckerrüben oder bei Obst, und eine gleichmäßigere Reife des Erntegutes, die zu höheren Erträgen führt.

Erhöhung der Widerstandskraft gegen klimatische Einflüsse wie Kälte und Trockenheit.

Beeinflussung des Latexflusses bei Gummipflanzen.

Ausbildung parthenokarper Früchte, Pollensterilität und Geschlechtsbeeinflussung sind ebenfalls Anwendungsmöglichkeiten.

Kontrolle der Keimung von Samen oder des Austriebs von Knospen.

Entlaubung oder Beeinflussung des Fruchtfalles zur Ernteerleichterung.

-4-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Die erfindungsgemäßen Stoffe entfalten ihre Wirkung sowohl bei Vor- als auch bei Nachauflaufbehandlung.

Sie eignen sich insbesondere zur Beeinflussung des vegetativen und generativen Wachstums bei Leguminosen, wie zum Beispiel Soja.

Die Aufwandmengen betragen je nach Anwendungsziel im allgemeinen von 0,005 bis 5 kg Wirkstoff/ha, können jedoch gegebenenfalls auch in höheren Aufwandmengen eingesetzt werden.

Die Anwendungszeit richtet sich nach dem Anwendungsziel und den klimatischen Bedingungen. Je nach Pflanzenart und Aufwandmenge können sich auch gewisse herbizide Effekte ergeben.

Die erfindungsgemäßen Verbindungen eignen sich überraschenderweise auch zur Erhöhung der Streßresistenz bei Kulturpflanzen wie Baumwolle, Buschbohne, Gurke, Mais, Soja und anderen.

Besonders hervorzuheben ist die Erhöhung der Trockenresistenz.

Dieser Effekt ist in sofern von Bedeutung als in wichtigen Anbaugebieten viele Kulturen unter großem Energieaufwand künstlich bewässert werden müssen. Schätzungen zufolge werden im Jahr 2000 ca. 200 Millionen ha Land bewässert.

Ein Konditionieren der Pflanzen in der Hinsicht, daß ihnen weniger Wasser zugeführt werden muß, oder daß sie über einige Tage hinweg Trockenperioden ohne Bewässerung überstehen, würde eine große Arbeits- und Materialersparnis bedeuten und Verlusten vorbeugen.

Darüber hinaus könnten auch Anbauflächen bebaut werden, die bisher aufgrund zu großer Trockenheit nicht bebaut wurden.

-5-

Die erfindungsgemäßen Verbindungen weisen außerdem eine bakterizide Wirkung auf.

Wegen des erkannten breiten Wirkungsspektrums eignen sich die Verbindungen nicht nur zum Schutz von Kulturpflanzen sondern auch zum Materialschutz und zur Bekämpfung humanpathogener und tierpathogener Mikroben, woraus sich breit gefächerte Anwendungsmöglichkeiten ergeben.

Die erfindungsgemäßen Verbindungen besitzen weiterhin die vorteilhafte Eigenschaft, Pflanzenarten in ihrer Entwicklung derart zu inhibieren, daß diese als Konkurrenz ausgeschaltet werden können. Dieser Effekt kann zur Unkrautbekämpfung benutzt werden.

Je nach der speziellen Bedeutung ihrer Substituenten ergeben sich bei den erfindungsgemäßen Verbindungen schwerpunktmäßige Anwendungsgebiete. So können diese jeweils als Fungizide, Pflanzenwachstumsregulatoren, Herbizide oder Bakterizide eingesetzt werden.

In den durch die allgemeine Formel I gekennzeichneten Verbindungen können zum Beispiel bedeuten:
$R_1$ Phenyl, Methylphenyl, Dimethylphenyl, Trimethylphenyl, Fluorphenyl, Difluorphenyl, Chlorphenyl, Dichlorphenyl, Trichlorphenyl, Bromphenyl, Dibromphenyl, Jodphenyl, Trifluormethylphenyl, Methoxyphenyl, Dimethoxyphenyl, Äthoxyphenyl, Methylthiophenyl, Äthylthiophenyl, Nitrophenyl, Cyanphenyl, Naphthyl, Pyridyl, Furyl und Thienyl,
Y ein N-Atom oder die CH-Gruppe,
Z die Carbonyl- oder CH(OH)-Gruppe,
$R_2$ und/oder $R_3$ Methyl, Äthyl, Propyl, n-Butyl, n-Pentyl, n-Hexyl, Isopropyl, Isobutyl, Cyclopropyl, Cyclopentyl, Cyclohexyl, 2-Propenyl, 2-Propinyl, Benzyl, Chlorbenzyl oder gemeinsam einen Alkylenrest

$$-(CH_2)_n-,$$

-6-

worin n die Zahlen 2, 3, 4, 5 oder 6 darstellt.

Die erfindungsgemäßen Verbindungen besitzen im Falle Z beziehungsweise Z' = Carbonyl und $R_1 \neq R_2$ zwei asymmetrische Kohlenstoffatome, im Falle Z beziehungsweise Z' = Carbonyl und $R_1 = R_2$ ein asymmetrisches Kohlenstoffatom. Sie können daher als maximal vier ($R_1 \neq R_2$) oder zwei ($R_1 = R_2$) räumliche Isomere vorliegen.
Im Falle Z beziehungsweise Z" = CH(OH) und $R_1 \neq R_2$ sind drei, im Falle Z beziehungsweise Z" = CH(OH) und $R_1 = R_2$ sind zwei asymmetrische Kohlenstoffatome vorhanden. Die Verbindungen können daher als maximal acht ($R_1 \neq R_2$) oder vier ($R_1 = R_2$) räumliche Isomere vorliegen. Diese Isomerengemische lassen sich durch an sich bekannte Verfahren, beispielsweise durch Chromatographie, in die einzelnen Isomeren auftrennen.

Alle Isomeren und deren Gemische sind Gegenstand der vorliegenden Erfindung.

Zur Bildung der Säureadditionssalze kommen sowohl anorganische Säuren in Frage, wie zum Beispiel Chlorwasserstoffsäure, Bromwasserstoffsäure, Salpetersäure, Phosphorsäure, Schwefelsäure, mono- und bifunktionelle Carbonsäuren und Hydroxycarbonsäuren, wie zum Beispiel Essigsäure, Maleinsäure, Oxalsäure, Bernsteinsäure, Fumarsäure, Weinsäure, Zitronensäure, Salizylsäure, Sorbinsäure, Milchsäure, sowie Sulfonsäuren, wie zum Beispiel p-Toluolsulfonsäure und 1,5-Naphthalindisulfonsäure.

Die Säureadditionssalze können nach den üblichen Salzbildungsverfahren, zum Beispiel durch Lösen einer Verbindung der Formel I in einem geeigneten Lösungsmittel und Hinzufügen der Säure erhalten werden.

Eine herausragende fungizide Wirkung zeigen zum Beispiel die folgenden erfindungsgemäßen Verbindungen:

-7-

2,2-Diäthyl-3-oxo-5-phenyl-4-(1,2,4-triazol-1-yl)-pentannitril

2,2-Diäthyl-3-oxo-5-phenyl-4-(imidazol-1-yl)-pentannitril

2,2-Dipropyl-3-oxo-5-phenyl-4-(1,2,4-triazol-1-yl)-pentannitril

2,2-Dipropyl--3-oxo-5-phenyl-4-(imidazol-1-yl)-pentannitril, Hydronitrat

2,2-Dipropyl-5-(4-fluorphenyl)-3-oxo-4-(1,2,4-triazol-1-yl)-pentannitril

2,2-Dipropyl-5-(4-fluorphenyl)-3-oxo-4-(1,2,4-triazol-1-yl)-pentannitril, Hydronitrat

2,2-Diäthyl-5-(4-fluorphenyl)-3-hydroxy-4-(1,2,4-triazol-1-yl)-pentannitril

2,2-Dipropyl-5-(4-fluorphenyl)-3-hydroxy-4-(1,2,4-triazol-1-yl)-pentannitril.

Die folgenden erfindungsgemäßen Verbindungen entfalten eine hervorragende pflanzenwachstumsregulatorische Wirkung.

5-(4-Chlorphenyl)-2,2-dimethyl-3-oxo-4-(1,2,4-triazol-1-yl)-pentannitril

5-(4-Chlorphenyl)-2,2-dimethyl-3-hydroxy-4-(1,2,4-triazol-1-yl)-pentannitril

2,2-Diäthyl-5-(4-fluorphenyl)-3-oxo-4-(1,2,4-triazol-1-yl)-pentannitril

2,2-Diäthyl-3-oxo-5-phenyl-4-(1,2,4-triazol-1-yl)-pentannitril, Hydronitrat

2,2-Diäthyl-3-oxo-5-phenyl-4-(1,2,4-triazol-1-yl)-pentannitril, Naphthalin 1,5-disulfonat

2,2-Dimethyl-3-oxo-5-phenyl-4-(1,2,4-triazol-1-yl)-pentannitril.

-8-

5-(2,4-Dichlorphenyl)-2,2-dimethyl-3-hydroxy-4-(imidazol-1-yl)-pentannitril

2,2-Diäthyl-3-oxo-5-phenyl-4-(1,2,4-triazol-1-yl)-pentannitril

2,2-Diäthyl-3-hydroxy-5-phenyl-4-(1,2,4-triazol-1-yl)-pentannitril

5-(4-Chlorphenyl)-2,2-dimethyl-3-oxo-4-(1,2,4-triazol-1-yl)-pentannitril, Hydronitrat

2,2-Dimethyl-3-oxo-5-phenyl-4-(1,2,4-triazol-1-yl)-pentannitril, Hydronitrat

5-(4-Chlorphenyl)-2,2-dimethyl-3-oxo-4-(1,2,4-triazol-1-yl)-pentannitril, Naphthalin-1,5-disulfonat

5-(4-Chlorphenyl)-2,2-diäthyl-3-oxo-4-(1,2,4-triazol-1-yl)-pentannitril, Naphthalin-1,5-disulfonat

2,2-Di-n-propyl-3-oxo-5-phenyl-4-(1,2,4-triazol-1-yl)-pentannitril, Hydronitrat

2,2-Di-n-propyl-3-oxo-5-phenyl-4-(imidazol-1-yl)-pentannitril, Hydronitrat

2,2-Dimethyl-3-oxo-5-phenyl-4-(1,2,4-triazol-1-yl)-pentannitril, Naphthalin-1,5-disulfonat

2,2-Di-n-propyl-5-(4-fluorphenyl)-3-oxo-4-(1,2,4-triazol-1-yl)-pentannitril

2,2-Dimethyl-5-(4-fluorphenyl)-3-hydroxy-4-(1,2,4-triazol-1-yl)-pentannitril

2,2-Dimethyl-5-(4-fluorphenyl)-3-hydroxy-4-(1,2,4-triazol-1-yl)-pentannitril, Hydronitrat

2,2-Diäthyl-5-(4-fluorphenyl)-3-oxo-4-(1,2,4-triazol-1-yl)-pentannitril, Hydronitrat

-9-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 1087006600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

2,2-Dimethyl-5-(4-fluorphenyl)-3-oxo-4-(1,2,4-triazol-1-yl)-pentannitril, Naphthalin-1,5-disulfonat

2,2-Dimethyl-3-hydroxy-5-(4-methylphenyl)-4-(1,2,4-triazol-1-yl)-pentannitril,

Zur Unkrautbekämpfung lassen sich zum Beispiel folgende erfindungsgemäße Verbindungen einsetzen:

5-(4-Chlorphenyl)-2,2-dimethyl-3-oxo-4-(1,2,4-triazol-1-yl)-pentannitril

5-(4-Chlorphenyl)-2,2-dimethyl-3-hydroxy-4-(1,2,4-triazol-1-yl)-pentannitril

5-(4-Chlorphenyl)-2,2-dimethyl-4-(imidazol-1-yl)-3-oxo-pentannitril.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden.

Sofern eine Verbreiterung des Wirkungsspektrums beabsichtigt ist, können auch andere Biozide zugesetzt werden. Beispielsweise eignen sich als herbizid wirksame Mischungspartner diejenigen Wirkstoffe, die in Weed Abstracts, Vol.31,No.7,1982, unter dem Titel "Lists of common names and abbreviations employed for currently used herbicides and plant growth regulators in weed abstracts" aufgeführt sind. Außerdem können auch nicht phytotoxische Mittel angewendet werden, die mit Herbiziden und/oder Wuchsregulatoren eine synergistische Wirkungssteigerung ergeben können, wie unter anderem Netzmittel, Emulgatoren, Lösungsmittel und ölige Zusätze.



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phasphatidylinosit, Phosphatidylserin, Lysolecithin und Phosphatidylglycerol.



Postanschrift: Schering Aktiengesellschaft, Postfach 65 03 11, D-1000 Berlin 65 · Für Besucher: Berlin-Wedding, Müllerstraße 170-178 Telegramme: Scheringchemie Be
Vorstand: Dr. Heribert Asmis, Dr. Christian Brunn, Dr. Heinz Hannse, Horst Kramp, Dr. Klaus Pohle, Dr. Horst Witzel Vorsitzender des Aufsichtsrats: Hans-Jürgen Ham:
Sitz der Gesellschaft: Berlin und Bergkamen Handelsregister: AG Charlottenburg 93 HRB 283 und AG Kamen HRB 0061 Berliner Commerzbank AG, Berlin, Konto 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto 2415008, Bankleitzahl 100 700 00 Postscheckamt Berlin West, Konto-Nr. 1175-101, Bankleitzahl 100 100 10

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Netz-, Haft-, Emulgier- und/oder Dispergierhilfsmitteln angewandt.

Geeignete flüssige Trägerstoffe sind zum Beispiel Wasser, aliphatische und aromatische Kohlenwasserstoffe, wie Benzol, Toluol, Xylol, Cyclohexanon, Isophoron, Dimethylsulfoxyd, Dimethylformamid, weiterhin Mineralölfraktionen und Pflanzenöle.

Als feste Trägerstoffe eignen sich Mineralerden, zum Beispiel Tonsil, Silicagel, Talkum, Kaolin, Attapulgit, Kalkstein, Kieselsäure und pflanzliche Produkte, zum Beispiel Mehle.

An oberflächenaktiven Stoffen sind zu nennen: zum Beispiel Calciumligninsulfonat, Polyoxyäthylenalkylphenyl-äther, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Sofern die Wirkstoffe zur Saatgutbeizung Verwendung finden sollen, können auch Farbstoffe zugemischt werden, um dem gebeizten Saatgut eine deutlich sichtbare Färbung zu geben.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozente flüssige oder feste Trägerstoffe sowie gegebenenfalls bis zu 20 Gewichtsprozente oberflächenaktive Stoffe.

-12-

Die Ausbringung der Mittel kann in üblicher Weise erfolgen, zum Beispiel mit Wasser als Träger in Spritzbrühmengen von etwa 100 bis 1000 Liter/ha. Eine Anwendung der Mittel im sogenannten "Low-Volume-" oder "Ultra-Low-Volume-Verfahren" ist ebenso möglich wie ihre Applikation in Form von sogenannten Mikrogranulaten.

Zur Herstellung der Zubereitungen werden zum Beispiel die folgenden Bestandteile eingesetzt:

A. Spritzpulver

a) 40 Gewichtsprozent Wirkstoff
25 Gewichtsprozent Tonmineralien
20 Gewichtsprozent Kieselsäure
10 Gewichtsprozent Zellpech
5 Gewichtsprozent oberflächenaktive Stoffe auf der Basis einer Mischung des Calciumsalzes der Ligninsulfonsäure mit Alkylphenolpolyglykoläthern

b) 25 Gewichtsprozent Wirkstoff
60 Gewichtsprozent Kaolin
10 Gewichtsprozent Kieselsäure
5 Gewichtsprozent oberflächenaktive Stoffe auf Basis des Natriumsalzes des N-Methyl-N-oleyl-taurins und des Calciumsalzes der Ligninsulfonsäure.

c) 10 Gewichtsprozent Wirkstoff
60 Gewichtsprozent Tonmineralien
15 Gewichtsprozent Kieselsäure
10 Gewichtsprozent Zellpech
5 Gewichtsprozent oberflächenaktive Stoffe auf Basis des Natriumsalzes des N-Methyl-N-oleyl-taurins und des Calciumsalzes der Ligninsulfonsäure.

-13-

B. Paste

45 Gewichtsprozent Wirkstoff
5 Gewichtsprozent Natriumaluminiumsilikat
15 Gewichtsprozent Cetylpolyglycoläther mit 8 Mol Äthylenoxid
2 Gewichtsprozent Spindelöl
10 Gewichtsprozent Polyäthylenglycol
23 Teile Wasser

C. Emulsionkonzentrat

25 Gewichtsprozent Wirkstoff
15 Gewichtsprozent Cyclohexanon
55 Gewichtsprozent Xylol
5 Gewichtsprozent Mischung von Nonylphenylpolyoxyäthylen oder Calciumdodecylbenzolsulfonat.

Die neuen erfindungsgemäßen Verbindungen lassen sich zum Beispiel herstellen, indem man Verbindungen der allgemeinen Formel

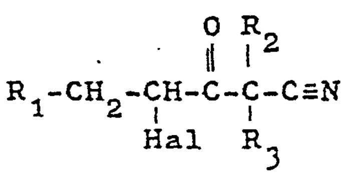

II

mit Verbindungen der allgemeinen Formel

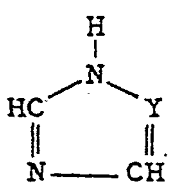

III

oder deren Metallsalzen in Gegenwart eines inerten Lösungsmittels und gegebenenfalls in Gegenwart eines Säureakzeptors unter Bildung von Verbindungen der allgemeinen Formel



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin

Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

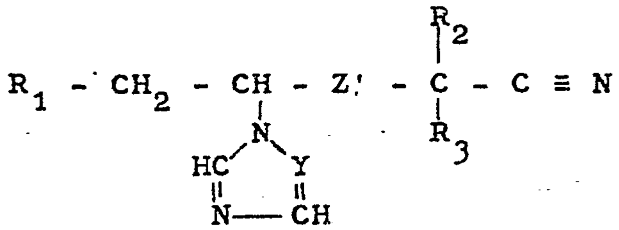

IV

umsetzt, die man gewünschtenfalls zu Verbindungen der allgemeinen Formel

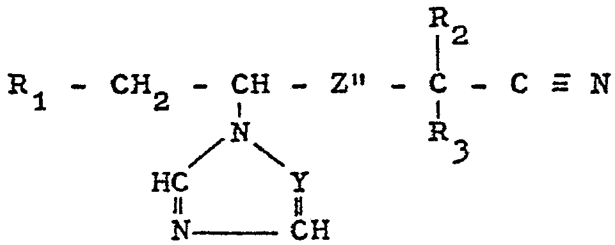

V

reduziert, worin $R_1$, $R_2$, $R_3$ und Y die oben genannte Bedeutung haben, Hal ein Halogenatom, vorzugsweise ein Bromatom,
Z' die Carbonylgruppe und Z" die CH(OH)-Gruppe bedeutet.

Die Umsetzung der Verbindungen der allgemeinen Formel II mit Verbindungen der allgemeinen Formel III erfolgt in Gegenwart eines inerten organischen Lösungsmittels, wie zum Beispiel Acetonitril oder Dimethylformamid und in Gegenwart eines Säureakzeptors, wie Kaliumcarbonat, Calciumcarbonat, Triäthylamin oder mit einem Überschuß der Verbindung der allgemeinen Formel III.

Setzt man die Metallsalze der Verbindungen der allgemeinen Formel III um, eigenen sich dafür besonders die Alkali- und Erdalkalimetallsalze. Man erzeugt diese vorzugsweise in situ durch Umsetzen von 1,2,4-Triazol oder Imidazol mit einem Erdalkali- oder Alkali-Hydrid, wie Lithiumhydrid oder Natriumhydrid oder einem Alkoholat, wie Natrium- oder Lithiummethylat, in einem geeigneten inerten organischen Lösungsmittel, wie Dimethylformamid oder Dimethylsulfoxid.

Zur Reduktion der Verbindungen der allgemeinen Formel IV verwendet man zweckmäßigerweise komplexe Metallhydride wie



Postanschrift: Schering Aktiengesellschaft, Postfach 65 03 11, D-1000 Berlin 65 · Für Besucher: Berlin-Wedding, Müllerstraße 170-178 Telegramme: Scheringchemie Be

Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Ham.
Sitz der Gesellschaft: Berlin und Bergkamen · Handelsregister: AG Charlottenburg 93 HRB 283 und AG Kamen HRB 0061 · Berliner Commerzbank AG, Berlin, Konto 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto 2415008, Bankleitzahl 100 700 00 · Postscheckamt Berlin West, Konto-Nr. 1175-101, Bankleitzahl 100 100 10

zum Beispiel Natriumborhydrid, oder Aluminiumalkoxide, wie zum Beispiel Aluminiumisopropylat. Bei Verwendung von Natriumborhydrid eignen sich als Lösungsmittel insbesondere Äther, wie Diäthyläther oder Tetrahydrofuran, Alkohole wie Methanol, Äthanol oder Isopropanol oder aromatische Kohlenwasserstoffe wie Toluol.

Die Reaktionstemperatur beträgt vorzugsweise -10°C bis +30°C. Führt man die Reduktion mittels Aluminiumisopropylat durch, werden als Lösungsmittel Alkohole, wie Isopropanol, oder aromatische Kohlenwasserstoffe, wie Toluol,eingesetzt.

Die erfindungsgemäßen Verbindungen sind im Falle,daß Z die Carbonylgruppe darstellt, fast farb- und geruchlose Öle oder kristalline Verbindungen oder im Falle, daß Z die CH(OH)-Gruppe darstellt, farb- und geruchlose kristalline Verbindungen. Alle diese Verbindungen lösen sich schlecht in Wasser und mehr oder weniger gut in organischen Lösungsmitteln, wie zum Beispiel Alkoholen, Äthern, Aceton oder chlorierten Kohlenwasserstoffen, gut hingegen in aprotischen Lösungsmitteln wie Dimethylformamid oder Dimethylsulfoxid.

Die Säureadditionssalze lösen sich zum Teil in Wasser und gut in polaren organischen Lösungsmitteln wie Acetonitril, Dimethylformamid und Dimethylsulfoxid.

Die folgenden Beispiele erläutern die Herstellung der erfindungsgemäßen Verbindungen.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis · Dr. Christian Brunn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101 Bankleitzahl 100 100 10

BEISPIEL 1

5-(4-Chlorphenyl)-2,2-dimethyl-4-(imidazol-1-yl)-3-oxo-pentannitril

6 g (0,12 Mol) ca. 50%iges Natriumhydrid werden in 100 ml Dimethylformamid suspendiert und unter Kühlung bei 15-20°C mit 8,2 g (0,12 Mol) Imidazol in 40 ml Dimethylformamid versetzt. Nach Beendigung der Wasserstoffentwicklung werden unter Kühlung bei 20-25°C 38 g (0,12 Mol) 4-Brom-5-(4-chlorphenyl)-2,2-dimethyl-3-oxo-pentannitril gelöst in 60 ml Dimethylformamid zugetropft. Es wird eine Stunde bei Raumtemperatur nachgerührt und anschließend auf 1,5 Liter Wasser gegeben. Die Wasserphase wird nach Neutralstellen mit reichlich Ammoniumchlorid ausgezalzen und mehrfach mit Methylenchlorid ausgeschüttelt. Nach dem Trocknen der organischen Phase mit Magnesiumsulfat wird im Vakuum eingeengt und an Kieselgel chromatographiert.

Ausbeute: farbloses Öl 20,3 g = 56,1 % der Theorie

| | | | | | |
|---|---|---|---|---|---|
| Analyse: | berechnet | C 63,67 % | H 5,34 % | N 13,92 % | Cl 11,74 % |
| | gefunden | C 63,03 % | H 5,59 % | N 13,27 % | Cl 12,12 % |



Postanschrift: Schering Aktiengesellschaft, Postfach 65 03 11, D-1000 Berlin 65 · Für Besucher: Berlin-Wedding, Müllerstraße 170-178 Telegramme: Scheringchemie B.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Ham
Sitz der Gesellschaft: Berlin und Bergkamen Handelsregister: AG Charlottenburg 93 HRB 283 und AG Kamen HRB 0061 · Berliner Commerzbank AG, Berlin Konto 108700600, Bankleitzahl 100 400 00 Berliner Handels- und Frankfurter Bank, Berlin, Konto-Nr 70045224, Bankleitzahl 100 202 00 · Deutsche Bank Berlin AG, Konto 2415008, Bankleitzahl 100 700 00 Postscheckamt Berlin West, Konto-Nr 1175-101, Bankleitzahl 100 100 10

BEISPIEL 2

5-(4-Chlorphenyl)-2,2-dimethyl-3-hydroxy-4-(imidazol-1-yl)-pentannitril

13 g (0,043 Mol) 5-(4-Chlorphenyl)-2,2-dimethyl-4-(imidazol-1-yl)-3-oxo-pentannitril werden in 100 ml Methanol gelöst und bei 0°C portionsweise mit 1,22 g (0,03 Mol) Natriumborhydrid versetzt und eine Stunde unter langsamer Erwärmung auf Raumtemperatur nachgerührt. Anschließend wird im Vakuum eingeengt, der Rückstand mit verdünnter Salzsäure verrührt, neutral gestellt und der Feststoff abgesaugt. Nach Umkristallisation aus Isopropanol erhält man weiße Kristalle.

| | | | | | |
|---|---|---|---|---|---|
| Ausbeute: | 5,4 g = 41 % der Theorie | | | | |
| Fp.: | 243-245°C | | | | |
| Analyse: | berechnet: | C 63,25 % | H 5,97 % | N 13,83 % | Cl 11,67 % |
| | gefunden : | C 62,95 % | H 6,20 % | N 13,70 % | Cl 11,21 % |

-18-

BEISPIEL 3

5-(4-Chlorphenyl)-2,2-dimethyl-3-oxo-4-(1,2,4-triazol-1-yl)-pentannitril

6 g (0,12 Mol) ca. 50%iges Natriumhydrid werden in 150 ml Dimethylformamid suspendiert und bei Raumtemperatur mit 8,6g (0,12 Mol) 1,2,4-Triazol in 40 ml Dimethylformamid versetzt. Nach Beendigung der Wasserstoffentwicklung werden 38 g (0,12 Mol) 4-Brom-5-(4-chlorphenyl)-2,2-dimethyl-3-oxo-pentannitril unter Rühren und Kühlen bei 25-30°C zugetropft. Man rührt 1 Stunde bei Raumtemperatur nach und gießt auf 1,5 Liter Wasser, säuert schwach an, salzt mit Ammoniumchlorid aus und extrahiert mehrmals mit Methylenchlorid. Nach dem Trocknen der organischen Phase über Magnesiumsulfat wird im Vakuum eingeengt und aus Diisopropyläther/Äther umkristallisiert.

| | | | | | |
|---|---|---|---|---|---|
| Ausbeute: | 18,3 g = 50,3 % der Theorie | | | | |
| Fp.: | 88-89°C | | | | |
| Analyse : | berechnet | C 59,50% | H 4,99% | N 18,50% | Cl 11,77% |
| | gefunden | C 59,60% | H 4,95% | N 18,21% | Cl 11,58% |

-19-

BEISPIEL 4

5-(4-Chlorphenyl)-2,2-dimethyl-3-hydroxy-4-(1,2,4-triazol-1-yl)-pentannitril

8,3 g (0,027 Mol) 5-(4-Chlorphenyl)-2,2-dimethyl-3-oxo-4-(1,2,4-triazol-1-yl)-pentannitril werden in 80 ml Methanol bei 0°C, vorgelegt und portionsweise mit 0,8 g (0,02 Mol) Natriumborhydrid versetzt. Nach einstündigem Rühren unter Erwärmen auf Raumtemperatur wird eingeengt und mit verdünnter Salzsäure hydrolysiert. Die nach dem Neutralstellen erhaltenen Kristalle werden aus Isopropanol umkristallisiert.

Ausbeute: 6,2 g = 75% der Theorie
Fp.: 193-195°C

| | | | | | |
|---|---|---|---|---|---|
| Analyse: | berechnet | C 59,11% | H 5,62% | N 18,38% | Cl 11,63% |
| | gefunden | C 59,50% | H 5,98% | N 18,18% | Cl 11,38% |

-20-

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 Berliner Commerzbank AG, Berlin Konto-Nr 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr 2415008, Bankleitzahl 100 700 00 Postscheckamt Berlin West, Konto-Nr 1175-101 Bankleitzahl 100 100 10

BEISPIEL 5

2,2-Diäthyl-5-(4-fluorphenyl)-3-oxo-4-(1,2,4-triazol-1-yl)-pentannitril

3,7 g (0,077 Mol) 50%iges Natriumhydrid werden bei 20°C in 100 ml Dimethylformamid suspendiert und mit 5,3 g (0,077 Mol) 1,2,4-Triazol, in 30 ml Dimethylformamid gelöst, versetzt. Nach Beendigung der Wasserstoffentwicklung werden 25 g (0,077 Mol) 4-Brom-2,2-diäthyl-5-(4-fluorphenyl)-3-oxo-pentannitril gelöst in 30 ml Dimethylformamid zugetropft. Nach zweistündigem Rühren bei 30°C wird auf ca. 1,5 Liter Wasser gegossen und nach Aussalzen mit Natriumchlorid mehrfach mit Methylenchlorid extrahiert. Die Methylenchloridphase wird mit Wasser zurückgewaschen und über Magnesiumsulfat getrocknet.

Rohausbeute nach dem Einengen im Vakuum: 23,3 g = 96% der Theorie

Das Rohprodukt wird in 200 ml Aceton gelöst und mit einer aretonischen Lösung von 19g Naphthalin-1,5-disulfonsäure versetzt. Die nach einiger Zeit ausfallenden Kristalle werden abgesaugt. Sie sind das Additionssalz der Titelverbindung mit Naphthalin-1,5-disulfonsäure.

Die freie Verbindung erhält man daraus durch Behandeln mit verdünnter Natronlauge und Methylenchlorid. Die organische Phase wird anschließend getrocknet und eingeengt. Man erhält das Produkt in Form eines schwach gefärbten Öles.

Ausbeute: 7,7 g = 32 % der Theorie

| | | | | | |
|---|---|---|---|---|---|
| Analyse: | berechnet | C 64,95% | H 6,09% | F 6,04% | N 17,82% |
| | gefunden | C 64,55% | H 5,89% | F 5,99% | N 18,23% |

-21-

BEISPIEL 6

2,2-Diäthyl-5-(4-fluorphenyl)-3-hydroxy-4-(1,2,4-triazol-1-yl)-pentannitril

3,7 g (0,0118Mol) 2,2-Diäthyl-5-(4-fluorphenyl)-3-oxo-4-(1,2,4-triazol-1-yl)-pentannitril werden in 40 ml Methanol gelöst und bei 0°C portionsweise mit 0,5 g (0,0118 Mol) Natriumborhydrid versetzt. Nach einstündigem Nachrühren unter Erwärmung auf Raumtemperatur wird auf Eis/Wasser gegeben und angesäuert. Anschließend wird wieder mit Natriumhydrogencarbonat neutralgestellt und mit Methylenchlorid extrahiert. Die organische Phase wird über Magnesiumsulfat getrocknet, im Vakuum eingeengt und mit Diisopropyläther kristallisiert.

Ausbeute: 2,8 g = 75% der Theorie
Fp.: 130-132°C

| | | | | | |
|---|---|---|---|---|---|
| Analyse: | berechnet | C 64,54% | H 6,69% | F 6,01% | N 17,71% |
| | gefunden | C 64,75% | H 6,65% | F 6,01% | N 17,46% |

-22-

BEISPIEL 7

2,2-Di-n-propyl-5-(4-fluorphenyl)-4-(imidazol-1-yl)-3-oxo-pentannitril

37,8 g (0,111 Mol) 4-Brom-5-(4-fluorphenyl)-2,2-di-n-propyl-3-oxo-pentannitril werden in 50 ml Dimethylformamid gelöst und bei 10 bis 15°C zu einer vorbereiteten Lösung von Imidazol-Natrium in Dimethylformamid getropft. Die Imidazol-Natrium Lösung wird folgendermaßen erhalten:
Zu einer Suspension von 5,3 g 50%igem Natriumhydrid (entsprechend 0,11 Mol) in 100 ml Dimethylformamid werden 7,6 g (0,111 Mol) Imidazol in 30ml Dimethylformamid bei ca. 10°C zugegeben und bis zum Ende der Wasserstoffentwicklung gerührt. Die gesamte Reaktionsmischung wird 2 Stunden bei Raumtemperatur gerührt und anschließend auf Eis/Wasser gegossen. Es wird mit Methylenchlorid extrahiert und die organische Phase mit wenig Wasser mehrmals zurückgewaschen.
Nach dem Trocknen der Methylenchloridphase über Magnesiumsulfat wird im Vakuum vollständig eingeengt und der ölige Rückstand an Kieselgel chromatografiert.

Ausbeute: 2,9 g = 7,7% der Theorie (Öl)

| | | | | | |
|---|---|---|---|---|---|
| Analyse: | berechnet | C 70,36% | H 7,59% | F 5,56% | N 12,31% |
| | gefunden | C 69,98% | H 7,43% | F 5,65% | N 12,07% |

-23-

## BEISPIEL 8

2,2-Di-n-propyl-5-(4-fluorphenyl)-3-hydroxy-4-(imidazol-1-yl)-pentannitril

1,9 g (0,0056 Mol) 2,2-Di-n-propyl-5-(4-fluorphenyl)-4-(imidazol-1-yl)-3-oxo-pentannitril werden in 30 ml Methanol gelöst und bei 0°C portionsweise mit 0,24 g (0,0056 Mol) Natriumborhydrid versetzt. Man läßt 2 Stunden bei Raumtemperatur nachrühren und versetzt mit 500 ml verdünnter Salzsäure. Nach 10 Minuten wird mit Natriumhydrogencarbonat neutralisiert. Die erhaltenen Kristalle werden abgesaugt und im Vakuum getrocknet.

Ausbeute: 1,4 g = 72,9 % der Theorie

Fp.: 180-182°C

| | | | | | |
|---|---|---|---|---|---|
| Analyse: | berechnet | C 69,94% | H 7,63% | F 5,53% | N 12,27% |
| | gefunden | C 69,73% | H 7,66% | F 5,69% | N 12,02% |



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 · Berliner Commerzbank AG Berlin Konto-Nr. 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

BEISPIEL 9

2,2-Diäthyl-3-oxo-5-phenyl-4-(1,2,4-triazol-1-yl)-pentannitril, Hydronitrat

1,6 g (0,0054 Mol) 2,2-Diäthyl-3-oxo-5-phenyl-4-(1,2,4-triazol-1-yl)-pentannitril werden in 20 ml Isopropanol gelöst und mit 0,25 ml (0,006 Mol) 100%iger Salpetersäure versetzt. Die nach dem Abkühlen auf 0°C ausgefallenen Kristalle werden abgesaugt und im Vakuum getrocknet.

| | | | | |
|---|---|---|---|---|
| Ausbeute: | 1,6 g = 82 % der Theorie | | | |
| Fp.: | 135°C (Zersetzung) | | | |
| Analyse: | berechnet | C 56,81% | H 5,89% | N 19,48% |
| | gefunden | C 56,99% | H 5,82% | N 19,43% |

-25-

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Brunn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG, Berlin, Konto-Nr. 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

BEISPIEL 10

2,2-Diäthyl-3-oxo-5-phenyl-4-(1,2,4-triazol-1-yl)-pentannitril, Naphthalin-1,5-disulfonat

1,6 g (0,0054 Mol) 2,2-Diäthyl-3-oxo-5-phenyl-4-(1,2,4-triazol-1-yl)-pentannitril werden in 20 ml Aceton gelöst und mit 1,4 g (0,0035 Mol) 72%iger Naphthalin-1,5-disulfonsäure in 20 ml Aceton versetzt. Die nach kurzem Erwärmen auf dem Dampfbad eintretende kristalline Fällung wird abgesaugt und im Vakuum getrocknet.

| | | | | | |
|---|---|---|---|---|---|
| Ausbeute: | 2,0 g = 84 % der Theorie | | | | |
| Fp.: | 245°C | | | | |
| Analyse: | berechnet | C 59,98% | H 5,49% | N 12,71% | S 7,27% |
| | gefunden | C 60,00% | H 5,33% | N 12,95% | S 7,58% |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 · Berliner Commerzbank AG, Berlin Konto-Nr 108700600, Bankleitzahl 100 400 00 Berliner Handels- und Frankfurter Bank, Berlin, Konto-Nr 70045224, Bankleitzahl 100 202 00 Deutsche Bank Berlin AG, Konto-Nr 2415008, Bankleitzahl 100 700 00 Postscheckamt Berlin West, Konto-Nr 1175-101, Bankleitzahl 100 100 10

In analoger Weise lassen sich die folgenden erfindungsgemäßen Verbindungen herstellen:

| Beispiel Nr. | Name der Verbindungen | Physikalische Konstante |
|---|---|---|
| 11 | 2,2-Dimethyl-3-oxo-5-phenyl-4-(1,2,4-triazol-1-yl)-pentannitril | Fp.: 87- 89°C |
| 12 | 2,2-Dimethyl-3-hydroxy-5-phenyl-4-(1,2,4-triazol-1-yl)-pentannitril | Fp.:154-156°C |
| 13 | 2,2-Dimethyl-4-(imidazol-1-yl)-3-oxo-5-phenyl-pentannitril | zähes Öl |
| 14 | 2,2-Dimethyl-3-hydroxy-4-(imidazol-1-yl)-5-phenylpentannitril | Fp.:186-188°C |
| 15 | 5-(2,4-Dichlorphenyl)-2,2-dimethyl-3-oxo-4-(1,2,4-triazol-1-yl)-pentannitril | Fp.:118-120°C |
| 16 | 5-(2,4-Dichlorphenyl)-2,2-dimethyl-3-hydroxy-4-(1,2,4-triazol-1-yl)-pentannitril | Fp.:200-208°C |
| 17 | 5-(2,4-Dichlorphenyl)-2,2-dimethyl-3-hydroxy-4-(imidazol-1-yl)-pentannitril | Fp.:211-212°C |
| 18 | 2,2-Diäthyl-3-oxo-5-phenyl-4-(1,2,4-triazol-1-yl)-pentannitril | zähes Öl |
| 19 | 2,2-Diäthyl-3-oxo-5-phenyl-4-(imidazol-1-yl)-pentannitril | zähes Öl |
| 20 | 2,2-Diäthyl-3-hydroxy-5-phenyl-4-(1,2,4-triazol-1-yl)-pentannitril | Fp.:117-118°C |
| 21 | 2,2-Di-n-propyl-3-oxo-5-phenyl-4-(1,2,4-triazol-1-yl)-pentannitril | zähes Öl |
| 22 | 5-(4-Chlorphenyl)-2,2-dimethyl-3-oxo-4-(1,2,4-triazol-1-yl)-pentannitril, Hydronitrat | Fp.:127°C (Zersetzung) |

-27-

| Beispiel Nr. | Name der Verbindungen | Physikalische Konstante |
|---|---|---|
| 23 | 2,2-Dimethyl-3-oxo-5-phenyl-4-(1,2,4-triazol-1-yl)-pentannitril, Hydronitrat | Fp.:125°C (Zersetzung) |
| 24 | 5-(2,4-Dichlorphenyl)-2,2-dimethyl-3-oxo-4-(1,2,4-triazol-1-yl)-pentannitril, Hydronitrat | Fp.:130°C (Zersetzung) |
| 25 | 5-(4-Chlorphenyl)-2,2-dimethyl-3-oxo-4-(1,2,4-triazol-1-yl)-pentannitril, Naphthalin-1,5-disulfonat | Fp.:247-249°C |
| 26 | 5-(4-Chlorphenyl)-2,2-diäthyl-3-hydroxy-4-(1,2,4-triazol-1-yl)-pentannitril | Fp.:163-167°C |
| 27 | 5-(4-Chlorphenyl)-2,2-diäthyl-3-oxo-4-(1,2,4-triazol-1-yl)-pentannitril, Naphthalin-1,5-disulfonat | Fp.:200°C |
| 28 | 2,2-Diäthyl-3-hydroxy-5-phenyl-4-(imidazol-1-yl)-pentannitril | Fp.:125-127°C |
| 29 | 2,2-Di-n-propyl-3-oxo-5-phenyl-4-(1,2,4-triazol-1-yl)-pentannitril, Naphthalin-1,5-disulfonat | Fp.:185-190°C |
| 30 | 2,2-Di-n-propyl-3-hydroxy-5-phenyl-4-(1,2,4-triazol-1-yl)-pentannitril | Fp.:125-126°C |
| 31 | 2,2-Di-n-propyl-3-oxo-5-phenyl-4-(imidazol-1-yl)-pentannitril | zähes Öl |
| 32 | 2,2-Di-n-propyl-3-hydroxy-5-phenyl-4-(imidazol-1-yl)-pentannitril | Fp.:157-158°C |
| 33 | 2,2,Di-n-propyl-3-hydroxy-5-phenyl-4-(imidazol-1-yl)-pentannitril, Hydronitrat | Fp.:105°C (Zersetzung) |
| 34 | 2,2-Di-n-propyl-3-oxo-5-phenyl-4-(1,2,4-triazol-1-yl)-pentannitril, Hydronitrat | Fp.:105-108°C (Zersetzung) |

-28-

| Beispiel Nr. | Name der Verbindung | Physikalische Konstante |
|---|---|---|
| 35 | 2,2-Di-n-propyl-3-oxo-5-phenyl-4-(imidazol-1-yl)-pentannitril, Hydronitrat | Fp.: 170 °C (Zersetzung) |
| 36 | 2,2-Diäthyl-3-oxo-5-phenyl-4-(imidazol-1-yl)-pentannitril, Hydronitrat | Fp.: 148 °C (Zersetzung) |
| 37 | 2,2-Dimethyl-3-oxo-5-phenyl-4-(1,2,4-triazol-1-yl)-pentannitril, Naphthalin-1,5-disulfonat | Fp.: 84 °C (Zersetzung) |
| 38 | 5-(2,4-Dichlorphenyl)-2,2-dimethyl-3-oxo-4-(1,2,4-triazol-1-yl)-pentannitril | Fp.: 209-210 °C |
| 39 | 2,2-Dimethyl-5-(4-fluorphenyl)-3-oxo-4-(1,2,4-triazol-1-yl)-pentannitril | Fp.: 108-110 °C |
| 40 | 2,2-Di-n-propyl-5-(4-fluorphenyl)-3-oxo-4-(1,2,4-triazol-1-yl)-pentannitril | zähes Öl |
| 41 | 2,2-Dimethyl-5-(4-fluorphenyl)-3-hydroxy-4-(1,2,4-triazol-1-yl)-pentannitril | Fp.: 152-155 °C |
| 42 | 2,2-Dimethyl-5-(4-fluorphenyl)-3-oxo-4-(1,2,4-triazol-1-yl)-pentannitril, Hydronitrat | Fp.: 135 °C (Zersetzung) |
| 43 | 2,2-Dimethyl-5-(4-fluorphenyl)-3-hydroxy-4-(1,2,4-triazol-1-yl)-pentannitril, Hydronitrat | Fp.: 180 °C (Zersetzung) |
| 44 | 2,2-Dimethyl-3-hydroxy-4-(imidazol-1-yl)-5-(4-methylphenyl)-pentannitril | Fp.: 180-182 °C |
| 45 | 2,2-Diäthyl-5-(4-fluorphenyl)-3-oxo-4-(1,2,4-triazol-1-yl)-pentannitril, Hydronitrat | Fp.: 118 °C (Zersetzung) |
| 46 | 2,2-Di-n-propyl-5-(4-fluorphenyl)-3-oxo-4-(1,2,4-triazol-1-yl)-pentannitril, Hydronitrat | Fp.: 125 °C (Zersetzung) |
| 47 | 2,2-Dimethyl-5-(4-fluorphenyl)-3-oxo-4-(1,2,4-triazol-1-yl)-pentannitril, Naphthalin-1,5-disulfonat | Fp.: 195-196 °C |



Postanschrift: Schering Aktiengesellschaft, Postfach 65 03 11, D-1000 Berlin 65 · Für Besucher Berlin-Wedding, Müllerstraße 170-178 Telegramme: Scheringchemie Ber

Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hama
Sitz der Gesellschaft: Berlin und Bergkamen Handelsregister: AG Charlottenburg 93 HRB 283 und AG Kamen HRB 0061 Berliner Commerzbank AG, Berlin, Konto-108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-2415008, Bankleitzahl 100 700 00 Postscheckamt Berlin West, Konto-Nr. 1175-101, Bankleitzahl 100 100 10

| Beispiel Nr. | Name der Verbindung | Physikalische Konstante |
|---|---|---|
| 48 | 2,2-Dimethyl-5-(4-fluorphenyl)-3-hydroxy-4-(1,2,4-triazol-1-yl)-pentannitril, Naphthalin-1,5-disulfonat | Fp.:160-162°C |
| 49 | 2,2-Diäthyl-5-(4-fluorphenyl)-3-oxo-4-(1,2,4-triazol-1-yl)-pentannitril, Naphthalin-1,5-disulfonat | Fp.:200°C |
| 50 | 2,2-Diäthyl-3-hydroxy-5-(4-methylphenyl)-4-(1,2,4-triazol-1-yl)-pentannitril | Fp.:123-125°C |
| 51 | 2,2-Di-n-propyl-5-(4-fluorphenyl)-3-hydroxy-4-(1,2,4-triazol-1-yl)-pentannitril | Fp.:145°C (Zersetzung) |
| 52 | 5-(4-Chlorphenyl)-2,2-diäthyl-3-oxo-4-(1,2,4-triazol-1-yl)-pentannitril | zähes Öl |
| 53 | 2,2-Dimethyl-5-(4-methylphenyl)-3-oxo-4-(1,2,4-triazol-1-yl)-pentannitril | Fp.: 65- 67°C |
| 54 | 5-(4-Chlorphenyl)-2,2-diäthyl-3-hydroxy-4-(1,2,4-triazol-1-yl)-pentannitril, Naphthalin-1,5-disulfonat | Fp.:200°C |
| 55 | 2,2-Dimethyl-3-hydroxy-5-(4-methylphenyl)-4-(1,2,4-triazol-1-yl)-pentannitril | Fp.:169-172°C |
| 56 | 2,2-Diäthyl-5-(4-methylphenyl)-3-oxo-4-(1,2,4-triazol-1-yl)-pentannitril | Fp.: 67- 70°C |
| 57 | 2,2-Dimethyl-4-(imidazol-1-yl)-5-(4-methylphenyl)-3-oxo-pentannitril | Fp.: 87- 90°C |
| 58 | 5-(2-Chlorphenyl)-2,2-dimethyl-3-oxo-4-(1,2,4-triazol-1-yl)-pentannitril | Fp.:101-102°C |
| 59 | 5-(2-Chlorphenyl)-2,2-dimethyl-3-hydroxy-4-(imidazol-1-yl)-pentannitril | Fp.:204-205°C |

-30-

| Beispiel Nr. | Name der Verbindung | Physikalische Konstante |
|---|---|---|
| 60 | 2,2-Di-n-propyl-5-(4-fluorphenyl)-4-(imidazol-1-yl)--3-oxo-pentannitril | Öl |
| 61 | 2,2-Diäthyl-5-(4-fluorphenyl)-3-hydroxy-4-(1,2,4-triazol-1-yl)-pentannitril, Naphthalin-1,5-disulfonat | Fp.: 158°C (Zersetzung) |
| 62 | 2,2-Diäthyl-5-(4-fluorphenyl)-3-hydroxy-4-(1,2,4-triazol-1-yl)-pentannitril, Hydronitrat | Fp.: 168°C (Zersetzung) |
| 63 | 2,2-Di-n-propyl-5-(4-fluor-phenyl)-3-hydroxy-4-(1,2,4-triazol-1-yl)-pentannitril, Naphthalin-1,5-disulfonat | Fp.: 210°C |
| 64 | 2,2-Di-n-propyl-5-(4-fluor-phenyl)-3-hydroxy-4-(1,2,4-triazol-1-yl)-pentannitril, Hydronitrat | Fp.: 122-124°C |
| 65 | 2,2-Diäthyl-5-(4-fluorphe-nyl)-3-hydroxy-4-(imidazol--1-yl)-pentannitril | Fp.: 172-174°C |
| 66 | 2,2-Di-n-propyl-5-(4-fluor-phenyl)-3-hydroxy-4-(imi-dazol-1-yl)-pentannitril | Fp.: 180-182°C |
| 67 | 2,2-Dimethyl-5-(2-methyl-phenyl)-3-oxo-4-(1,2,4-triazol-1-yl)-pentannitril | Fp.: 93- 94°C |
| 68 | 2,2-Dimethyl-5-(2-methyl-phenyl)-3-hydroxy-4-(1,2,4-triazol-1-yl)-pentannitril | Fp.: 168-170°C |
| 69 | 2,2-Diäthyl-5-(2,4-dichlor-phenyl)-3-oxo-4-(1,2,4-triazol-1-yl)-pentannitril | Fp.: 90- 92°C |
| 70 | 2,2-Diäthyl-5-(2,4-dichlor-phenyl)-3-oxo-4-(imidazol--1-yl)-pentannitril | Fp.: 131-133°C |
| 71 | 2,2-Diäthyl-5-(2,4-dichlor-phenyl)-3-hydroxy-4-(imi-dazol-1-yl)-pentannitril | Fp.: 162-164°C |
| 72 | 2,2-Diäthyl-5-(2,4-dichlor-phenyl)-3-hydroxy-4-(1,2,4-triazol-1-yl)-pentannitril | Fp.: 139-140°C |

-31-

| Beispiel Nr. | Name der Verbindung | Physukalische Konstante |
|---|---|---|
| 73 | 5-(2-Chlorphenyl)-2,2-dimethyl-3-hydroxy-4-(1,2,4-triazol-1-yl)-pentannitril | Fp.:147-149°C |
| 74 | 5-(2-Chlorphenyl)-2,2-dimethyl-3-oxo-4-(1,2,4-triazol-1-yl)-pentannitril, Hydronitrat | Fp.: 133°C (Zersetzung) |
| 75 | 5-(2-Chlorphenyl)-2,2-dimethyl-3-oxo-4-(imidazol-1-yl)-pentannitril | Öl |
| 76 | 5-(2-Chlorphenyl)-2,2-diäthyl-3-oxo-4-(1,2,4-triazol-1-yl)-pentannitril | Fp.: 74- 76°C |
| 77 | 5-(2-Chlorphenyl)-2,2-diäthyl-3-hydroxy-4-(1,2,4-triazol-1-yl)-pentannitril | Fp.:122-124°C |
| 78 | 5-(4-Chlorphenyl)-2,2-di-n-propyl-3-oxo-4-(1,2,4-triazol-1-yl)-pentannitril | Fp.: 83- 84°C |
| 79 | 5-(4-Chlorphenyl)-2,2-di-n-propyl-3-oxo-4-(1,2,4-triazol-1-yl)-pentannitril, Hydronitrat | Fp.: 146°C (Zersetzung) |
| 80 | 5-(4-Chlorphenyl)-2,2-di-n-propyl-4-(imidazol-1-yl)-3-oxo-pentannitril | Öl |
| 81 | 5-(4-Chlorphenyl)-2,2-di-n-propyl-3-hydroxy-4-(imidazol-1-yl)-pentannitril | Fp.:146-148°C |
| 82 | 5-(4-Chlorphenyl)-2,2-di-n-propyl-3-hydroxy-4-(1,2,4-triazol-1-yl)-pentannitril | Fp.: 148°C (Zersetzung) |
| 83 | 3-Oxo-2,2-pentamethylen-5-phenyl-4-(1,2,4-triazol-1-yl)-pentannitril | Fp.:138-140°C |
| 84 | 3-Hydroxy-2,2-pentamethylen-5-phenyl-4-(1,2,4-triazol-1-yl)-pentannitril | Fp.:142-143°C |

-32-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Beispiel Nr. | Name der Verbindung | Physikalische Konstante |
|---|---|---|
| 85 | 5-(4-Chlorphenyl)-3-oxo--2,2-pentamethylen-4-(1,2,4-triazol-1-yl)-pentannitril | Fp.:136-138°C |
| 86 | 5-(2,4-Dichlorphenyl)-3--oxo-2,2-pentamethylen-4--(1,2,4-triazol-1-yl)-pentannitril | Fp.:107-109 |
| 87 | 5-(4-Chlorphenyl)-3-hydroxy--2,2-pentamethylen-4-(1,2,4-triazol-1-yl)-pentannitril | Fp.:140-142°C |
| 88 | 5-(2,4-Dichlorphenyl)-3-hydroxy-2,2-pentamethylen--4-(1,2,4-triazol-1-yl)-pentannitril | Fp.:191-193°C |
| 89 | 2,2-Di-n-butyl-5-(2,4-dichlorphenyl)-3-oxo-4-(1,2,4-triazol-1-yl)-pentannitril | Fp.:100-101°C |
| 90 | 5-(2-Chlorphenyl)-2,2-di-n-propyl-3-oxo-4-(1,2,4-triazol-1-yl)-pentannitril | Fp.: 88- 90°C |
| 91 | 5-(2-Chlorphenyl)-2,2-di-n-propyl-3-hydroxy-4-(1,2,4-triazol-1-yl)-pentannitril | Fp.:147-149°C |
| 92 | 5-(2,4-Dichlorphenyl)-2,2-di-n-butyl-3-hydroxy-4-(1,2,4-triazol-1-yl)-pentannitril | Fp.: 95- 96°C |
| 93 | 5-(2,4-Dichlorphenyl)-2,2-di-n-propyl-3-oxo-4-(1,2,4-triazol-1-yl)-pentannitril | Fp.: 95- 97°C |
| 94 | 5-(2,4-Dichlorphenyl)-2,2-di-n-propyl-3-hydroxy-4-(1,2,4-triazol-1-yl)-pentannitril | Fp.: 91- 92°C |

-33-

Postanschrift: Schering Aktiengesellschaft, Postfach 65 03 11, D-1000 Berlin 65 · Für Besucher: Berlin-Wedding, Müllerstraße 170-178 Telegramme: Scheringchemie B
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Har
Sitz der Gesellschaft: Berlin und Bergkamen · Handelsregister: AG Charlottenburg 93 HRB 283 und AG Kamen HRB 0061 Berliner Commerzbank AG, Berlin, Kont
108700600, Bankleitzahl 100 400 00 Berliner Handels- und Frankfurter Bank, Berlin, Konto-Nr. 70045224, Bankleitzahl 100 202 00 Deutsche Bank Berlin AG, Kont
2415008, Bankleitzahl 100 700 00 Postscheckamt Berlin West, Konto-Nr 1175-101, Bankleitzahl 100 100 10

Die Ausgangsprodukte zur Herstellung der erfindungsgemäßen Verbindungen sind an sich bekannt oder können nach an sich bekannten Verfahren hergestellt werden.

Die Ausgangsverbindungen der allgemeinen Formel II erhält man durch Halogenieren von ß-Ketonitrilen der allgemeinen Formel

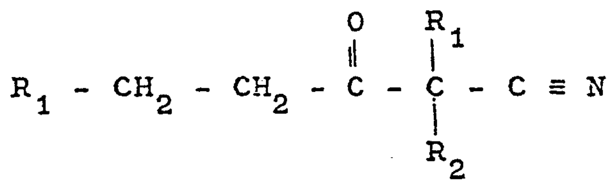

VI

mit Halogenierungsmitteln, wie beispielsweise Brom, Chlor oder Sulfurylchlorid. Das bevorzugte Verfahren besteht in der Umsetzung der Verbindungen der allgemeinen Formel VI mit elementarem Brom in geeigneten Lösungsmitteln, wie aliphatischen Carbonsäuren, zum Beispiel Essigsäure, oder in Äthern wie zum Beispiel Tetrahydrofuran oder Diäthyläther, und einem sauren Katalysator, wie zum Beispiel Bortrifluorid-Ätherat oder konzentrierter Schwefelsäure.

Die Ausgangsverbindungen zur Herstellung der Verbindungen der allgemeinen Formel VI werden durch doppelte C-Alkylierung der ß-Keto-Nitrile der allgemeinen Formel

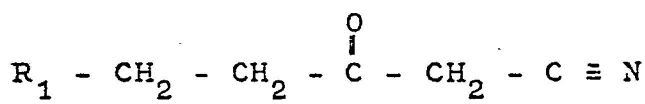

VII

nach an sich bekannten Verfahren dargestellt.

Im Falle der Umsetzung von Verbindungen der allgemeinen Formel VII zu Verbindungen der allgemeinen Formel VI mit untereinander verbundenen Resten $R_1$ und $R_2$ wird zur Alkylierung eine Verbindung der allgemeinen Formel VIII

$$X - (CH_2)_n - X \qquad \text{VIII}$$

verwendet, wobei X Brom, Jod, den Tosyl- oder den Mesylrest

-34-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis · Dr. Christian Brunn · Dr. Heinz Hannse · Horst Kramp · Dr. Klaus Pohle · Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

darstellt und n die Zahlen 2, 3, 4, 5 oder 6 darstellt.

Die Alkylierung wird gegebenenfalls in Gegenwart eines Lösungsmittels durchgeführt. Als Lösungsmittel können Alkohole, wie zum Beispiel Methanol, Äthanol, Isopropanol, und Äther, wie zum Beispiel Tetrahydrofuran oder Diäthyläther, aromatische Kohlenwasserstoffe, wie zum Beispiel Toluol oder Xylole, und vorzugsweise aprotische Lösungsmittel, wie zum Beispiel Dimethylformamid oder Dimethylsulfoxid,eingesetzt werden.

Als Basen kommen anorganische und organische Reagentien in Betracht, wie zum Beispiel Alkali- und Erdalkali-Hydroxide, -Oxide, -Carbonate und -Hydride oder tert.-Amine, wie z. B. Triäthylamin, N,N-Dimethylanilin, oder metallorganische Basen, wie zum Beispiel Natrium-, Lithium- oder Aluminium-organische Verbindungen, beispielsweise Lithiumphenyl, Lithiummethyl, n-Butyllithium und Trimethylaluminium.

Die Alkylierung erfolgt in einem der erwähnten Lösungsmittel mit einer der Basen bei Temperaturen vorzugsweise von 0 bis 100°C.

Ein weiteres Verfahren zur Herstellung der ß-Keto-Nitrile der allgemeinen Formel VII ist die Umsetzung der Hydrozimtsäureester der allgemeinen Formel

$$R_1 - CH_2 - CH_2 - \overset{O}{\overset{\|}{C}} - O - R' \qquad IX$$

in der $R_1$ die oben genannte Bedeutung hat und R' für einen Alkyl-, Aralkyl- oder Phenyl-Rest, vorzugsweise für einen Methyl- oder Äthylrest, steht, mit Acetonitril.

Die Umsetzung des Acetonitrils mit den Verbindungen der allgemeinen Formel IXerfolgt zweckmäßigerweise in Gegenwart einer Base, vorzugsweise Natriumhydrid.



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG, Berlin, Konto-Nr. 108700600, Bankleitzahl 100 400 00 Berliner Handels- und Frankfurter Bank, Berlin, Konto-Nr. 70045224 Bankleitzahl 100 202 00 Deutsche Bank Berlin AG, Konto-Nr. 2415008, Bankleitzahl 100 700 00 Postscheckamt Berlin West Konto-Nr. 1175-101 Bankleitzahl 100 100 10

Die folgenden Beispiele dienen zur Erläuterung der Herstellung der Ausgangsverbindungen.

4-Brom-5-(4-chlorphenyl)-2,2-dimethyl-3-oxo-pentannitril

5-(4-Chlorphenyl)-3-oxo-pentannitril

In sechs gleichen Ansätzen werden jeweils 19,2 g (0,4 Mol) 50%iges Natriumhydrid in 250 ml absolutem Tetrahydrofuran suspendiert und zum Sieden unter Rückfluß erwärmt. Dazu wird langsam eine Lösung bestehend aus 84,4 g (0,4 Mol) 3-(4-Chlorphenyl)-propionsäureäthylester (=4'-Chlor-hydrozimtsäureäthylester) und 18 g (0,44 Mol) Acetonitril in 100 ml Tetrahydrofuran zugetropft. Nach 2-stündigem Sieden wird mit 5 ml Methanol und anschließend mit 600 ml Hexan versetzt.

Nach dem Erkalten wird das Natriumsalz abgesaugt und die vereinigten trockenen Kristallisate auf 8 Liter Wasser gegeben und filtriert. Das Filtrat wird mit Salzsäure angesäuert und der sich abscheidende Feststoff scharf abgesaugt und im Vakuum getrocknet.

Ausbeute: 339,3 g = 68% der Theorie
Fp.: 83-84°C



Postanschrift: Schering Aktiengesellschaft, Postfach 65 03 11, D-1000 Berlin 65 · Für Besucher: Berlin-Wedding, Müllerstraße 170-178 Telegramme Scheringchemie Berlin
Vorstand: Dr. Herbert Asmis, Dr. Christian Brunn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG Berlin Konto-Nr. 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008, Bankleitzahl 100 700 00 Postscheckamt Berlin West Konto-Nr. 1175-101, Bankleitzahl 100 100 10

5-(4-Chlorphenyl)-2,2-dimethyl-3-oxo-pentannitril

120 g (0,57 Mol) der Vorstufe: 5-(4-Chlorphenyl)-3-oxo-pentannitril werden in 1,2 Liter Dimethylformamid gelöst und bei 0°C mit 124,2 g (2,3 Mol) Natriummethylat versetzt. Anschließend tropft man bei 0 bis 10°C 326,4 g (2,3 Mol) Jodmethan zu und rührt dann 1,5 Stunden bei Raumtemperatur nach. Anschließend wird die Hälfte bis 2/3 des Dimethylformamids im Vakuum entfernt und der Rückstand mit 2 Liter Wasser versetzt und mit Methylenchlorid mehrmals extrahiert. Die vereinigten Methylenchloridphasen werden mit Wasser zurückgewaschen und über Magnesiumsulfat getrocknet. Nach dem Einengen im Vakuum wird das erhaltene Öl bei 0,2 Torr fraktioniert destilliert.

Ausbeute: 86,5 g = 64% der Theorie
Kp.: 130-135°C/0,2 Torr



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 · Berliner Commerzbank AG, Berlin, Konto-Nr 1087000600, Bankleitzahl 100 400 00 · Berliner Handels- und Frankfurter Bank, Berlin, Konto-Nr 70045224, Bankleitzahl 100 202 00 · Deutsche Bank Berlin AG, Konto-Nr 2415008, Bankleitzahl 100 700 00 · Postscheckamt Berlin West, Konto-Nr 1175-101 Bankleitzahl 100 100 10

4-Brom-5-(4-chlorphenyl)-2,2-dimethyl-3-oxo-pentannitril

86,5 g (0,37 Mol) der Vorstufe: 5-(4-Chlorphenyl)-2,2-dimethyl-3-oxo-pentannitril werden in 500 ml trockenem Äther gelöst und mit 5,8 g konzentrierter Schwefelsäure versetzt.

Dann werden bei 30°C 58,6 g (0,36 Mol) Brom zugegeben und 3 Stunden gerührt. Nach weiterer Zugabe von 23,4 g Brom wird nochmals 2 Stunden gerührt und mit Eiswasser versetzt. Die Ätherphase wird getrocknet, eingeengt, mit Hexan aufgenommen, filtriert und nochmals im Vakuum bis zur Trockne eingeengt.
Es werden 113 g = 98% eines Öls erhalten.

-38-

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0051 Berliner Commerzbank AG, Berlin Konto-Nr. 108700600 Bankleitzahl 100 400 00 Berliner Handels- und Frankfurter Bank, Berlin Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

4-Brom-2,2-diäthyl-5-(4-fluorphenyl)-3-oxo-pentannitril

5-(4-Fluorphenyl)-3-oxo-pentannitril

In 3 gleichen Ansätzen wird jeweils eine Mischung bestehend aus 98,12 g (0,5 Mol) 3-(4-Fluorphenyl)-propionsäureäthylester, 26,7 g (0,65 Mol) Acetonitril und 30 ml Tetrahydrofuran zu einer siedenden Suspension aus 24 g 50%igem Natriumhydrid (ca. 0,5 Mol) in 200 ml Tetrahydrofuran langsam eingetropft. Nachdem die Wasserstoffentwicklung nahezu zum Stillstand gekommen ist wird noch 2 Stunden am Rückfluß gekocht. Anschließend wird mit 5 ml Methanol und 200 ml Hexan versetzt. Das ausgefallene Natriumsalz des 5-(5-Fluorphenyl)-3-oxo-pentannitrils wird scharf abgesaugt und aus allen drei Ansätzen vereinigt.
Man gibt in 1,5 Liter Eiswasser und säuert die Lösung mit Salzsäure an. Der Niederschlag wird abgesaugt und im Vakuum bei 40°C getrocknet.

Gesamtausbeute: 185 g = 64,5% der Theorie
Fp.: 80-83°C

| | | | | | |
|---|---|---|---|---|---|
| Analyse: | berechnet | C 69,03% | H 5,27% | F 9,94% | N 7,33% |
| | gefunden | C 69,00% | H 5,19% | F 9,69% | N 6,64% |

-39-

Postanschrift: Schering Aktiengesellschaft, Postfach 65 03 11, D-1000 Berlin 65 · Für Besucher: Berlin-Wedding, Müllerstraße 170-178 Telegramme Scheringchemie Berlin
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 1087006000 Bankleitzahl 100 400 00 · Berliner Handels- und Frankfurter Bank Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West, Konto-Nr. 1175-101, Bankleitzahl 100 100 10

2,2-Diäthyl-5-(4-fluorphenyl)-3-oxo-pentannitril

60 g* 5-(4-Fluorphenyl)-3-oxo-pentannitril werden in 200 ml Dimethylformamid gelöst und mit etwa einem Drittel von 9,4g 90%igem Lithiumhydrid (entsprechend 1,05 Mol) portionsweise versetzt. Man hält die Temperatur bei 20°C und versetzt nach nahezu beendeter Wasserstoffentwicklung mit 195,9 g (1,256 Mol) Äthyljodid. Anschließend werden die restlichen 2/3 des Lithiumhydrids portionsweise eingetragen. Man rührt 2 Stunden bei 50°C nach. Anschließend wird vorsichtig mit 1,5 Liter Eiswasser versetzt und überschüssiges Lithiumhydrid zerstört. Dann wird mit Methylenchlorid extrahiert, die organische Phase mit Magnesiumsulfat getrocknet und im Vakuum eingeengt. Der Rückstand wird im Vakuum destilliert.

* (0,314 Mol)

Ausbeute: 41,0 g = 52,8 % der Theorie

Kp.: 120°C/0,1 Torr

-40-

Postanschrift: Schering Aktiengesellschaft, Postfach 65 03 11, D-1000 Berlin 65 · Für Besucher: Berlin-Wedding, Müllerstraße 170-178 Telegramme: Scheringchemie Be

Vorstand: Dr. Herbert Asmis Dr. Christian Bruhn, Dr. Heinz Hannse, Horst Kramp, Dr. Klaus Pohle, Dr. Horst Witzel Vorsitzender des Aufsichtsrats: Hans-Jürgen Hamm Sitz der Gesellschaft: Berlin und Bergkamen Handelsregister: AG Charlottenburg 93 HRB 283 und AG Kamen HRB 0061 Berliner Commerzbank AG Berlin Konto-Nr. 108700600, Bankleitzahl 100 400 00 Berliner Handels- und Frankfurter Bank, Berlin, Konto-Nr. 70045224 Bankleitzahl 100 202 00 Deutsche Bank Berlin AG Konto-2415008 Bankleitzahl 100 700 00 Postscheckamt Berlin West, Konto-Nr. 1175-101, Bankleitzahl 100 100 10

4-Brom-2,2-diäthyl-5-(4-fluorphenyl)-3-oxo-pentannitril

41,0 g (0,166 Mol) 2,2-Diäthyl-5-(4-fluorphenyl)-3-oxo-pentannitril werden in 300 ml trockenem Äther gelöst und mit 2,66 g konzentrierter Schwefelsäure versetzt. Dann wird auf 30°C erwärmt und 26,5 g (0,166 Mol) Brom werden langsam zugetropft. Nach 1,5 Stunden war die Lösung entfärbt und man versetzt nochmals mit 8,0 g Brom (Überschuß). Nach einer Stunde wurde mit Eis und Wasser versetzt und die Ätherphase mehrmals mit Wasser gewaschen, getrocknet und eingeengt. Der ölige Rückstand wird in Hexan aufgenommen, filtriert und zur Trockne eingeengt.

Es werden 53,8 g = 99% der Theorie eines Öles erhalten.

Die folgenden Ausführungsbeispiele dienen zur Erläuterung der Anwendungsmöglichkeiten der erfindungsgemäßen Verbindungen, die in Form der oben angeführten Zubereitungen erfolgte.

Die Beispiele 95 bis 105 belegen die gute Wirkung der erfindungsgemäßen Verbindungen gegen pflanzenparasitäre Schadpilze unterschiedlichster Stellung im Pilzsystem.

In ppm = Teilen pro million Teile Spritzflüssigkeit ist die Anwendungskonzentration der Wirkstoffe angegeben.
Aus dem jeweils gefundenen Befall wurde die Fungizidwirkung in den nachfolgenden Beispielen wie folgt berechnet:

$$100 - \frac{\text{Befall in Behandelt} \cdot 100}{\text{Befall in Unbehandelt}} = \%\ \text{Wirkung}$$

In den angewandten fungitoxischen Konzentrationen waren die erfindungsgemäßen Verbindungen für die Kulturpflanzen verträglich.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 95

Wirkung prophylaktischer Blattbehandlung gegen Plasmopara viticola an Weinrebenpflanzen im Gewächshaus.

Junge Weinrebenpflanzen mit etwa 5 bis 8 Blättern wurden mit den angegebenen Konzentrationen tropfnaß gespritzt, nach Antrocknen des Spritzbelages mit einer wäßrigen Aufschwemmung von Sporangien des Pilzes (etwa 55.000 pro ml) blattunterseits besprüht und sofort im Gewächshaus bei 22 bis 24°C und möglichst wasserdampfgesättigter Atmosphäre inkubiert.

Vom zweiten Tage an wurde die Luftfeuchtigkeit für 3 bis 4 Tage auf Normalhöhe zurückgenommen (30 bis 70% Sättigung) und dann für einen Tag auf Wasserdampfsättigung gehalten. Anschließend wurde von jedem Blatt der prozentuale Anteil pilzbefallener Fläche notiert und der Durchschnitt je Behandlung zur Ermittlung der Fungizidwirkung wie oben beschrieben verrechnet.

-42-

| Erfindungsgemäße Verbindungen | % Wirkung 750 ppm | 250 ppm |
|---|---|---|
| 2,2-Diäthyl-3-oxo-5-phenyl--4-(imidazol-1-yl)-pentannitril | 96 | 83 |
| 2,2-Diäthyl-3-hydroxy-5--phenyl-4-(1,2,4-triazol--1-yl)-pentannitril | 100 | 96 |
| 2,2-Di-n-propyl-3-oxo-5--phenyl-4-(1,2,4-triazol--1-yl)-pentannitril | 100 | 100 |
| 5-(4-Chlorphenyl)-2,2--dimethyl-3-oxo-4-(1,2,4--triazol-1-yl)-pentannitril, Hydronitrat | 100 | 95 |
| 2,2-Dimethyl-3-oxo-5-phenyl--4-(1,2,4-triazol-1-yl)--pentannitril, Hydronitrat | 98 | 93 |
| 2,2-Diäthyl-3-oxo-5-phenyl--4-(1,2,4-triazol-1-yl)--pentannitril | 100 | 88 |
| 2,2-Di-n-propyl-3-oxo-5--phenyl-4-(imidazol-1-yl)--pentannitril, Hydronitrat | 100 | 97 |
| 2,2-Di-n-propyl-5-(4-fluorphenyl)-3-oxo-4-(1,2,4-triazol--1-yl)-pentannitril | 100 | 92 |
| 2,2-Di-n-propyl-5-(4-fluorphenyl)-3-oxo-4-(1,2,4--triazol-1-yl)-pentannitril, Hydronitrat | 92 | 80 |
| 2,2-Diäthyl-5-(4-fluorphenyl)-3-hydroxy-4-(1,2,4-triazol--1-yl)-pentannitril | 92 | 80 |
| **Vergleichsmittel gemäß DE-OS 2 734 442** | | |
| 1-(2,4-Dichlorphenyl)-4,4-dimethyl-2-(1,2,4-triazol-1-yl)-3-pentanol | | 0 |

-43-

## BEISPIEL 96

Wirkung prophylaktischer Spritzbehandlung gegen Phytophthora infestans an Tomatenpflanzen

Junge Tomatenpflanzen mit mindestens zwei gut entwickelten Laubblättern wurden tropfnaß gespritzt. Nach Antrocknen der Spritzbeläge wurden die behandelten Pflanzen sowie unbehandelte Kontrollpflanzen mit einer wäßrigen, 2 Stunden bei 11°C inkubierten Suspension von etwa 80 000 Phytophthora-Sporangien pro Milliliter besprüht. Bei etwa 18°C wurden die Pflanzen im Gewächshaus feucht inkubiert. Nach 5 Tagen wurde der prozentuale Anteil befallener Blattfläche notiert. Die Befunde sind nachfolgend angegeben. Das Vergleichsmittel hatte keine Wirkung.

| Erfindungsgemäße Verbindungen | % Wirkung mit 250 ppm |
|---|---|
| 5-(4-Chlorphenyl)-2,2-dimethyl-4-(imidazol-1-yl)-3-oxo-pentannitril | 85 |
| 5-(2,4-Dichlorphenyl)-2,2-dimethyl-3-oxo-4-(1,2,4-triazol-1-yl)-pentannitril | 70 |
| **Vergleichsmittel gemäß DE-OS 2 734 442** | |
| 1-(2,4-Dichlorphenyl)-4,4-dimethyl-2-(1,2,4-triazol-1-yl)-3-pentanol | 0 |

-44-

Postanschrift: Schering Aktiengesellschaft, Postfach 65 03 11, D-1000 Berlin 65 · Für Besucher: Berlin-Wedding, Müllerstraße 170-178 Telegramme Scheringchemie Berlin
Vorstand: Dr. Herbert Asmis, Dr. Christian Bruhn, Dr. Heinz Hannse, Horst Krämer, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG Berlin Konto-Nr. 108700600 Bankleitzahl 100 400 00 · Berliner Handels- und Frankfurter Bank Berlin Konto-Nr. 70045224, Bankleitzahl 100 202 00 · Deutsche Bank Berlin AG Konto-Nr. 2415008 Bankleitzahl 100 700 00 · Postscheckamt Berlin West Konto-Nr. 1175-101 Bankleitzahl 100 100 10

BEISPIEL 97

Wirkung prophylaktischer Spritzbehandlung gegen Botrytis cinerea an Tomatenpflanzen

Junge Tomatenpflanzen mit mindestens zwei gut entwickelten Laubblättern wurden mit der angegebenen Wirkstoffkonzentration tropfnaß gespritzt. Nach Antrocknen des Spritzbelages wurden die behandelten Pflanzen sowie unbehandelte Kontrollpflanzen inokuliert durch Aufsprühen einer Suspension von etwa 1 Million Botrytis-Sporen pro Milliliter Fruchtsaftlösung. Anschließend wurden die Pflanzen feucht bei etwa 20°C im Gewächshaus inkubiert. Nach dem Zusammenbrechen der unbehandelten Pflanzen (= 100% Befall) wurde der Befallsgrad der behandelten Pflanzen festgestellt.

| Erfindungsgemäße Verbindungen | % Wirkung mit 250 ppm |
|---|---|
| 2,2-Diäthyl-3-oxo-5-phenyl-4-(imidazol-1-yl)-pentannitril | 70 |
| 2,2-Diäthyl-3-hydroxy-5-phenyl-4-(1,2,4-triazol-1-yl)-pentannitril | 60 |
| 2,2-Di-n-propyl-3-oxo-5-phenyl-4-(1,2,4-triazol-1-yl)-pentannitril | 60 |
| 2,2-Di-n-propyl-3-hydroxy-5-phenyl-4-(1,2,4-triazol-1-yl)-pentannitril | 60 |
| 2,2-Di-n-propyl-3-oxo-5-phenyl-4-(imidazol-1-yl)-pentannitril | 80 |
| 2,2-Diäthyl-3-oxo-5-phenyl-4-(imidazol-1-yl)-pentannitril, Hydronitrat | 60 |



Postanschrift: Schering Aktiengesellschaft, Postfach 65 03 11, D-1000 Berlin 65 · Für Besucher: Berlin-Wedding, Müllerstraße 170-178 Telegramme Scheringchemie Be
Vorstand Dr. Herbert Asmis, Dr. Christian Bruhn, Dr. Heinz Hannse, Horst Kramp, Dr. Klaus Pohle, Dr. Horst Witzel · Vorsitzender des Aufsichtsrats Hans-Jürgen Hamm
Sitz der Gesellschaft Berlin und Bergkamen, Handelsregister: AG Charlottenburg 93 HRB 283 und AG Kamen HRB 0061 · Berliner Commerzbank AG Berlin, Konto-
108700600 Bankleitzahl 100 400 00 · Berliner Handels- und Frankfurter Bank, Berlin Konto-Nr. 70045224, Bankleitzahl 100 202 00 · Deutsche Bank Berlin AG, Konto-
2415008 Bankleitzahl 100 700 00 · Postscheckamt Berlin West, Konto-Nr. 1175-101, Bankleitzahl 100 100 10

| Erfindungsgemäße Verbindungen | % Wirkung mit 250 ppm |
|---|---|
| 2,2-Diäthyl-5-(2,4-dichlorphenyl)-3-oxo-4-(1,2,4-triazol-1-yl)-pentannitril | 85 |
| 2,2-Diäthyl-5-(2,4-dichlorphenyl)-3-oxo-4-(imidazol-1-yl)-pentannitril | 70 |
| 2,2-Diäthyl-5-(2,4-dichlorphenyl)-3-hydroxy-4-(imidazol-1-yl)-pentannitril | 80 |
| 2,2-Diäthyl-5-(2,4-dichlorphenyl)-3-hydroxy-4-(1,2,4-triazol-1-yl)-pentannitril | 75 |

-46-

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Heribert Asmis, Dr. Christian Brunn, Dr. Heinz Hannse, Horst Kramp, Dr. Klaus Pohle, Dr. Horst Witzel Vorsitzender des Aufsichtsrats: Hans-Jürgen Hamann Sitz der Gesellschaft: Berlin und Bergkamen Handelsregister: AG Charlottenburg 93 HRB 283 und AG Kamen HRB 0061 Berliner Commerzbank AG, Berlin Konto-Nr. 108700600, Bankleitzahl 100 400 00 Berliner Handels- und Frankfurter Bank, Berlin Konto-Nr. 70045224 Bankleitzahl 100 202 00 Deutsche Bank Berlin AG Konto-Nr. 2415008 Bankleitzahl 100 700 00 Postscheckamt Berlin West, Konto-Nr. 1175-101, Bankleitzahl 100 100 10

BEISPIEL 98

Wirkung prophylaktischer Spritzbehandlung gegen Piricularia oryzae an Reispflanzen

Junge Reispflanzen wurden mit der angegebenen Wirkstoffkonzentration tropfnaß gespritzt. Nach Antrocknen des Spritzbelages wurden die behandelten Pflanzen sowie unbehandelte Kontrollpflanzen durch Aufsprühen einer Piriculariasporen-Suspension (etwa 200 000 pro Milliliter) inokuliert und feucht bei 25 bis 27°C im Gewächshaus inkubiert. Nach 5 Tagen wurde der prozentuale Anteil befallener Blattfläche festgestellt.

| Erfindungsgemäße Verbindungen | % Wirkung mit | |
|---|---|---|
| | 500 ppm | 100 ppm |
| 2,2-Di-n-propyl-5-(4-fluorphenyl)-3-oxo-4-(1,2,4-triazol-1-yl)-pentannitril | 98 | 80 |
| 2,2-Diäthyl-3-oxo-5-phenyl-4-(1,2,4-triazol-1-yl)-pentannitril | 68 | |
| 2,2-Di-n-propyl-3-oxo-5-phenyl-4-(1,2,4-triazol-1-yl)-pentannitril, Naphthalin-1,5-disulfonat | 68 | |

-47-

Postanschrift: Schering Aktiengesellschaft, Postfach 65 03 11, D-1000 Berlin 65 Für Besucher: Berlin-Wedding, Müllerstraße 170-178 Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel. Vorsitzender des Aufsichtsrats: Hans-Jürgen Hamann. Sitz der Gesellschaft: Berlin und Bergkamen. Handelsregister: AG Charlottenburg 93 HRB 283 und AG Kamen HRB 0061. Berliner Commerzbank AG, Berlin, Konto-Nr. [illegible] Bankleitzahl 100 400 00. Berliner Handels- und Frankfurter Bank, Berlin, Konto-Nr. 70045224, Bankleitzahl 100 202 00. Deutsche Bank Berlin AG, Konto-Nr. [illegible] Bankleitzahl 100 700 00. Postscheckamt Berlin West, Konto-Nr. [illegible] Bankleitzahl 100 100 10

BEISPIEL 99

Wirkung prophylaktischer Spritzbehandlung gegen Cochliobolus miyabeanus an Reispflanzen

Junge Reispflanzen im Stadium des beginnenden zweiten Blattes wurden mit der angegebenen Konzentration tropfnaß gespritzt. Nach Antrocknen des Spritzbelages wurden die behandelten Pflanzen sowie unbehandelte Kontrollpflanzen mit einer wäßrigen Konidiensuspension (etwa 250 000 pro Milliliter) des Pilzes besprüht und im Gewächshaus bei 24°C feucht inkubiert. Nach 4 Tagen notierte man den prozentualen Anteil befallener Blattfläche.

| Erfindungsgemäße Verbindungen | % Wirkung mit 500 ppm |
|---|---|
| 2,2-Diäthyl-3-hydroxy-5-phenyl-4-(1,2,4-triazol-1-yl)-pentannitril | 68 |
| 5-(4-Chlorphenyl)-2,2-dimethyl-3-oxo-4-(1,2,4-triazol-1-yl)-pentannitril, Hydronitrat | 78 |
| 2,2-Diäthyl-3-hydroxy-5-phenyl-4-(imidazol-1-yl)-pentannitril | 68 |
| 2,2-Di-n-propyl-3-hydroxy-5-phenyl-4-(1,2,4-triazol-1-yl)-pentannitril | 80 |

-48-

BEISPIEL 100

Wirkung prophylaktischer Spritzbehandlung gegen Helminthosporium teres (Pyrenophora teres) an Gerste

Junge Gerstenpflanzen im Stadium des ersten Blattes wurden mit der angegebenen Konzentration tropfnaß gespritzt. Nach Antrocknen des Spritzbelages wurden die behandelten Pflanzen sowie unbehandelte Kontrollpflanzen mit einer wäßrigen Konidiosporensuspension (etwa 50 000 pro Milliliter) von Helminthosporium teres besprüht und in einer Feuchtkammer im Gewächshaus 2 Tage bei 20 bis 22 °C inkubiert. Danach wurden die Pflanzen im Gewächshaus bei 20 bis 22 °C kultiviert. Eine Woche nach der Inokulation wurde der prozentuale Anteil befallener Blattfläche notiert.

| Erfindungsgemäße Verbindungen | % Wirkung mit 500 ppm |
|---|---|
| 2,2-Dimethyl-3-hydroxy-5-phenyl-4-(1,2,4-triazol-1-yl)-pentannitril | 68 |
| 2,2-Dimethyl-4-(imidazol-1-yl)-3-oxo-5-phenyl-pentannitril | 84 |
| 5-(4-Chlorphenyl)-2,2-dimethyl-4-(imidazol-1-yl)-3-oxo-pentannitril | 68 |
| 2,2-Diäthyl-3-hydroxy-5-phenyl-4-(1,2,4-triazol-1-yl)-pentannitril | 100 |
| 2,2-Di-äthyl-3-oxo-5-phenyl-4-(1,2,4-triazol-1-yl)-pentannitril | 100 |
| 2,2-Di-äthyl-4-(imidazol-1-yl)-3-oxo-5-phenyl-pentannitril | 100 |
| 2,2-Di-n-propyl-3-oxo-5-phenyl-4-(1,2,4-triazol-1-yl)-pentannitril | 100 |
| 5-(4-Chlorphenyl)-2,2-dimethyl-3-oxo-4-(1,2,4-triazol-1-yl)-pentannitril, Hydronitrat | 100 |

-49-

Postanschrift: Schering Aktiengesellschaft, Postfach 65 03 11, D-1000 Berlin 65 · Für Besucher: Berlin-Wedding, Müllerstraße 170-178 Telegramme Scheringchemie
Vorstand: Dr. Heribert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen ... · Sitz der Gesellschaft: Berlin und Bergkamen · Handelsregister: AG Charlottenburg 93 HRB 283 und AG Kamen HRB 0061 · Berliner Commerzbank AG Berlin ... Bankleitzahl 100 400 00 · Berliner Handels- und Frankfurter Bank Berlin Konto-Nr. 70045224 Bankleitzahl 100 202 00 · Deutsche Bank Berlin AG ... Bankleitzahl 100 700 00 · Postscheckamt Berlin West Konto-Nr. 1175-101 Bankleitzahl 100 100 10

| Erfindungsgemäße Verbindungen | % Wirkung mit 500 ppm |
|---|---|
| 2,2-Dimethyl-3-oxo-5-phenyl-4-(1,2,4-triazol-1-yl)-pentannitril, Hydronitrat | 100 |
| 5-(2,4-Dichlorphenyl)-2,2-dimethyl-3-oxo-4-(1,2,4-triazol-1-yl)-pentannitril, Hydronitrat | 100 |
| 5-(4-Chlorphenyl)-2,2-dimethyl-3-oxo-4-(1,2,4-triazol-1-yl)-pentannitril, Naphthalin-1,5-disulfonat | 100 |
| 5-(4-Chlorphenyl)-2,2-diäthyl-3-hydroxy-4-(1,2,4-triazol-1-yl)-pentannitril | 98 |
| 5-(4-Chlorphenyl)-2,2-diäthyl-3-oxo-4-(1,2,4-triazol-1-yl)-pentannitril, Naphthalin-1,5-disulfonat | 89 |
| 2,2-Diäthyl-3-hydroxy-5-phenyl-4-(imidazol-1-yl)-pentannitril | 100 |
| 2,2-Di-n-propyl-3-oxo-5-phenyl-4-(1,2,4-triazol-1-yl)-pentannitril, Naphthalin-1,5-disulfonat | 100 |
| 2,2-Di-n-propyl-3-hydroxy-5-phenyl-4-(1,2,4-triazol-1-yl)-pentannitril | 96 |
| 2,2-Di-n-propyl-3-oxo-5-phenyl-4-(imidazol-1-yl)-pentannitril | 85 |
| 2,2-Di-n-propyl-3-hydroxy-5-phenyl-4-(imidazol-1-yl)-pentannitril | 79 |
| 2,2,Di-n-propyl-3-hydroxy-5-phenyl-4-(imidazol-1-yl)-pentannitril, Hydronitrat | 79 |
| 2,2-Di-n-propyl-3-oxo-5-phenyl-4-(1,2,4-triazol-1-yl)-pentannitril, Hydronitrat | 85 |



Postanschrift: Schering Aktiengesellschaft, Postfach 65 03 11, D-1000 Berlin 65 · Für Besucher: Berlin-Wedding, Müllerstraße 170-178 Telegramme Scheringchemie
Vorstand: Dr. Herbert Asmis, Dr. Christian Brunn, Dr. Heinz Hannse, Horst Kramp, Dr. Klaus Pohle, Dr. Horst Witzel Vorsitzender des Aufsichtsrats: Hans-Jürgen ...
Sitz der Gesellschaft: Berlin und Bergkamen Handelsregister: AG Charlottenburg 93 HRB 283 und AG Kamen HRB 0061 Berliner Commerzbank AG Berlin Kto
108700600 Bankleitzahl 100 400 00 Berliner Handels- und Frankfurter Bank Berlin Konto-Nr. 70045224 Bankleitzahl 100 202 00 Deutsche Bank Berlin AG ...

| Erfindungsgemäße Verbindungen | % Wirkung mit 500 ppm |
|---|---|
| 2,2-Di-n-propyl-3-oxo-5-phenyl-4-(imidazol-1-yl)-pentannitril, Hydronitrat | 89 |
| 2,2-Diäthyl-3-oxo-5-phenyl-4-(imidazol-1-yl)-pentannitril, Hydronitrat | 96 |
| 2,2-Dimethyl-3-oxo-5-phenyl-4-(1,2,4-triazol-1-yl)-pentannitril, Naphthalin-1,5-disulfonat | 79 |
| 5-(2,4-Dichlorphenyl)-2,2-dimethyl-3-oxo-4-(1,2,4-triazol-1-yl)-pentannitril | 89 |
| 2,2-Diäthyl-3-oxo-5-phenyl-4-(1,2,4-triazol-1-yl)-pentannitril, Hydronitrat | 79 |
| 2,2-Diäthyl-3-oxo-5-phenyl-4-(1,2,4-triazol-1-yl)-pentannitril, Naphthalin-1,5-disulfonat | 85 |
| 2,2-Dimethyl-5-(4-fluorphenyl)-3-oxo-4-(1,2,4-triazol-1-yl)-pentannitril | 100 |
| 2,2-Diäthyl-5-(4-fluorphenyl)-3-oxo-4-(1,2,4-triazol-1-yl)-pentannitril | 85 |
| 2,2-Di-n-propyl-5-(4-fluorphenyl)-3-oxo-4-(1,2,4-triazol-1-yl)-pentannitril | 92 |
| 2,2-Dimethyl-5-(4-fluorphenyl)-3-hydroxy-4-(1,2,4-triazol-1-yl)-pentannitril | 85 |
| 2,2-Dimethyl-5-(4-fluorphenyl)-3-oxo-4-(1,2,4-triazol-1-yl)-pentannitril, Hydronitrat | 92 |
| 2,2-Dimethyl-5-(4-fluorphenyl)-3-hydroxy-4-(1,2,4-triazol-1-yl)-pentannitril, Hydronitrat | 78 |
| 2,2-Diäthyl-5-(4-fluorphenyl)-3-oxo-4-(1,2,4-triazol-1-yl)-pentannitril, Hydronitrat | 92 |
| 2,2-Di-n-propyl-5-(4-fluorphenyl)-3-oxo-4-(1,2,4-triazol-1-yl)-pentannitril, Hydronitrat | 100 |

-51-

| Erfindungsgemäße Verbindungen | % Wirkung mit 500 ppm |
|---|---|
| 2,2-Dimethyl-5-(4-fluorphenyl)--3-oxo-4-(1,2,4--triazol-1-yl)-pentannitril, Naphthalin-1,5-disulfonat | 92 |
| 2,2-Dimethyl-5-(4-fluorphenyl)-3-hydroxy-4-(1,2,4--triazol-1-yl)-pentannitril, Naphthalin-1,5-disulfonat | 92 |
| 2,2-Diäthyl-5-(4-fluorphenyl)-3-oxo-4-(1,2,4--triazol-1-yl)-pentannitril, Naphthalin-1,5-disulfonat | 92 |
| 2,2-Diäthyl-5-(4-fluorphenyl)-3-hydroxy-4-(1,2,4-triazol--1-yl)-pentannitril | 97 |
| 2,2-Di-n-propyl-5-(4-fluorphenyl)-3-hydroxy-4-(1,2,4--triazol-1-yl)-pentannitril | 100 |

-52-

## BEISPIEL 101

Wirkung prophylaktischer Spritzbehandlung gegen Gerstenmehltau(Erysiphe graminis)

Junge Gerstenpflanzen im Stadium des ersten Blattes wurden mit angegebener Konzentration tropfnaß gespritzt, Nach Antrocknen des Spritzbelages wurden die behandelten Pflanzen sowie unbehandelte Kontrollpflanzen durch Überstreichen mit befallenen Pflanzen trocken inokuliert. Bei 20 bis 22°C wurden die Versuchspflanzen im Gewächshaus aufgestellt und nach einer Woche auf prozentualen Anteil befallener Blattfläche gemustert.

| Erfindungsgemäße Verbindungen | % Wirkung mit 500 | 250 | 100 ppm |
|---|---|---|---|
| 2,2-Dimethyl-3-oxo-5-phenyl-4-(1,2,4-triazol-1-yl)-pentannitril | 80 | | - |
| 2,2-Dimethyl-3-hydroxy-5-phenyl-4-(1,2,4-triazol-1-yl)-pentannitril | 100 | | 70 |
| 2,2-Dimethyl-4-(imidazol-1-yl)-3-oxo-5-phenyl-pentannitril | 100 | | 100 |
| 2,2-Dimethyl-3-hydroxy-4-(imidazol-1-yl)-5-phenylpentannitril | 100 | | 100 |
| 5-(4-Chlorphenyl)-2,2-dimethyl-3-oxo-4-(1,2,4-triazol-1-yl)-pentannitril | 100 | | 100 |
| 5-(4-Chlorphenyl)-2,2-dimethyl-3-hydroxy-4-(1,2,4-triazol-1-yl)-pentannitril | 100 | | 100 |
| 5-(4-Chlorphenyl)-2,2-dimethyl-4-(imidazol-1-yl)-3-oxo-pentannitril | 100 | | 100 |
| 5-(4-Chlorphenyl)-2,2-dimethyl-3-hydroxy-4-(imidazol-1-yl)-pentannitril | 70 | | - |



Postanschrift: Schering Aktiengesellschaft, Postfach 65 03 11, D-1000 Berlin 65 · Für Besucher: Berlin-Wedding, Müllerstraße 170-178 Telegramme Scheringchemie Berlin
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats Hans-Jürgen Hamann
Sitz der Gesellschaft: Berlin und Bergkamen Handelsregister: AG Charlottenburg 93 HRB 283 und AG Kamen HRB 0061 Berliner Commerzbank AG Berlin, Konto-Nr. 108700600, Bankleitzahl 100 400 00 Berliner Handels- und Frankfurter Bank Berlin, Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

| Erfindungsgemäße Verbindungen | % Wirkung mit 500 | 250 | 100 ppm |
|---|---|---|---|
| 5-(2,4-Dichlorphenyl)-2,2-dimethyl-3-oxo-4-(1,2,4-triazol-1-yl)-pentannitril | 100 | | 100 |
| 5-(2,4-Dichlorphenyl)-2,2-dimethyl-3-hydroxy-4-(1,2,4-triazol-1-yl)-pentannitril | 85 | | 75 |
| 5-(2,4-Dichlorphenyl)-2,2-dimethyl-3-hydroxy-4-(imidazol-1-yl)-pentannitril | 95 | | 90 |
| 2,2-Diäthyl-3-oxo-5--phenyl-4-(1,2,4-triazol--1-yl)-pentannitril | 100 | | 100 |
| 2,2-Di-äthyl-3-oxo-5--phenyl-4-(1,2,4-triazol--1-yl)-pentannitril | 100 | | 100 |
| 2,2-Di-äthyl-3-hydroxy-5-phenyl--4-(1,2,4-triazol-1-yl)-pentannitril | 100 | | 99 |
| 2,2-Di-n-propyl-3-oxo-5--phenyl-4-(1,2,4-triazol--1-yl)-pentannitril | 100 | | 99 |
| 5-(4-Chlorphenyl)-2,2--dimethyl-3-oxo-4-(1,2,4--triazol-1-yl)-pentannitril, Hydronitrat | 100 | | 99 |
| 2,2-Dimethyl-3-oxo-5-phenyl--4-(1,2,4-triazol-1-yl)--pentannitril, Hydronitrat | 100 | | 94 |
| 5-(2,4-Dichlorphenyl)-2,2-dimethyl-3-oxo-4-(1,2,4-triazol--1-yl)-pentannitril, Hydronitrat | 100 | | 99 |
| 5-(4-Chlorphenyl)-2,2-dimethyl-3-oxo-4-(1,2,4-triazol--1-yl)-pentannitril, Naphthalin-1,5-disulfonat | 100 | | 100 |
| 5-(4-Chlorphenyl)-2,2-diäthyl-3-hydroxy-4-(1,2,4--triazol-1-yl)-pentannitril | 99 | | 99 |
| 5-(4-Chlorphenyl)-2,2-diäthyl-3-oxo-4-(1,2,4-triazol--1-yl)-pentannitril, Naphthalin-1,5-disulfonat | 100 | | 100 |



Postanschrift: Schering Aktiengesellschaft, Postfach 65 03 11, D-1000 Berlin 65 · Für Besucher: Berlin-Wedding, Müllerstraße 170-178 Telegramme Scheringchemie B
Vorstand: Dr. Heribert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Ham
Sitz der Gesellschaft: Berlin und Bergkamen Handelsregister: AG Charlottenburg 93 HRB 283 und AG Kamen HRB 0061 Berliner Commerzbank AG Berlin Konto 108700600, Bankleitzahl 100 400 00 Berliner Handels- und Frankfurter Bank, Berlin, Konto-Nr. 70045224 Bankleitzahl 100 202 00 Deutsche Bank Berlin AG, Konto 2415008, Bankleitzahl 100 700 00 Postscheckamt Berlin West Konto-Nr. 1175-101 Bankleitzahl 100 100 10

| Erfindungsgemäße Verbindungen | % Wirkung mit 500 | 250 | 100 ppm |
|---|---|---|---|
| 2,2-Diäthyl-3-hydroxy-5-phenyl-4-(imidazol-1-yl)-pentannitril | 100 | | 94 |
| 2,2-Di-n-propyl-3-oxo-5-phenyl-4-(1,2,4-triazol-1-yl)-pentannitril, Naphthalin-1,5-disulfonat | 100 | | 94 |
| 2,2-Di-n-propyl-3-hydroxy-5-phenyl-4-(1,2,4-triazol-1-yl)-pentannitril | 100 | | 99 |
| 2,2-Di-n-propyl-3-oxo-5-phenyl-4-(imidazol-1-yl)-pentannitril | 100 | | 98 |
| 2,2-Di-n-propyl-3-hydroxy-5-phenyl-4-(imidazol-1-yl)-pentannitril | 95 | | - |
| 2,2,Di-n-propyl-3-hydroxy-5-phenyl-4-(imidazol-1-yl)-pentannitril, Hydronitrat | 95 | | 89 |
| 2,2-Di-n-propyl-3-oxo-5-phenyl-4-(1,2,4-triazol-1-yl)-pentannitril, Hydronitrat | 100 | | 98 |
| 2,2-Di-n-propyl-3-oxo-5-phenyl-4-(imidazol-1-yl)-pentannitril, Hydronitrat | 100 | | 95 |
| 2,2-Diäthyl-3-oxo-5-phenyl-4-(imidazol-1-yl)-pentannitril, Hydronitrat | 100 | | 98 |
| 2,2-Dimethyl-3-oxo-5-phenyl-4-(1,2,4-triazol-1-yl)-pentannitril, Naphthalin-1,5-disulfonat | 100 | | 100 |
| 5-(2,4-Dichlorphenyl)-2,2-dimethyl-3-oxo-4-(1,2,4-triazol-1-yl)-pentannitril | 100 | | 98 |
| 2,2-Diäthyl-3-oxo-5-phenyl-4-(1,2,4-triazol-1-yl)-pentannitril | 100 | | 100 |
| 2,2-Diäthyl-3-oxo-5-phenyl-4-(1,2,4-triazol-1-yl)-pentannitril, Naphthalin-1,5-disulfonat | 100 | | 100 |
| 2,2-Dimethyl-5-(4-fluorphenyl)-3-oxo-4-(1,2,4-triazol-1-yl)-pentannitril | 100 | | 100 |

-55-

| Erfindungsgemäße Verbindungen | % Wirkung mit 500 | 250 | 100 ppm |
|---|---|---|---|
| 2,2-Diäthyl-5-(4-fluorphenyl)-3-oxo-4-(1,2,4-triazol-1-yl)-pentannitril | 100 | | 100 |
| 2,2-Di-n-propyl-5-(4-fluorphenyl)-3-oxo-4-(1,2,4-triazol-1-yl)-pentannitril | 100 | | 100 |
| 2,2-Dimethyl-5-(4-fluorphenyl)-3-hydroxy-4-(1,2,4-triazol-1-yl)-pentannitril | 100 | | 100 |
| 2,2-Dimethyl-5-(4-fluorphenyl)-3-oxo-4-(1,2,4-triazol-1-yl)-pentannitril, Hydronitrat | 100 | | 100 |
| 2,2-Dimethyl-5-(4-fluorphenyl)-3-hydroxy-4-(1,2,4-triazol-1-yl)-pentannitril, Hydronitrat | 100 | | 100 |
| 2,2-Diäthyl-5-(4-fluorphenyl)-3-oxo-4-(1,2,4-triazol-1-yl)-pentannitril, Hydronitrat | 100 | | 100 |
| 2,2-Di-n-propyl-5-(4-fluorphenyl)-3-oxo-4-(1,2,4-triazol-1-yl)-pentannitril, Hydronitrat | 100 | | 100 |
| 2,2-Dimethyl-5-(4-fluorphenyl)--3-oxo-4-(1,2,4-triazol-1-yl)-pentannitril, Naphthalin-1,5-disulfonat | 100 | | 100 |
| 2,2-Dimethyl-5-(4-fluorphenyl)-3-hydroxy-4-(1,2,4-triazol-1-yl)-pentannitril, Naphthalin-1,5-disulfonat | 100 | | 100 |
| 2,2-Diäthyl-5-(4-fluorphenyl)-3-oxo-4-(1,2,4-triazol-1-yl)-pentannitril, Naphthalin-1,5-disulfonat | 100 | | 100 |
| 2,2-Diäthyl-5-(4-fluorphenyl)-3-hydroxy-4-(1,2,4-triazol-1-yl)-pentannitril | 100 | | 100 |
| 2,2-Di-n-propyl-5-(4-fluorphenyl)-3-hydroxy-4-(1,2,4-triazol-1-yl)-pentannitril | 100 | | 100 |

-56-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie B.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamm
Sitz der Gesellschaft: Berlin und Bergkamen · Handelsregister: AG Charlottenburg 93 HRB 283 und AG Kamen HRB 0061 · Berliner Commerzbank AG, Berlin, Konto 108700600, Bankleitzahl 100 400 00 · Berliner Handels- und Frankfurter Bank, Berlin, Konto-Nr. 7004524, Bankleitzahl 100 202 00 · Deutsche Bank Berlin AG, Konto 2415008, Bankleitzahl 100 700 00 · Postscheckamt Berlin West, Konto-Nr. 1175-101, Bankleitzahl 100 100 10

| Erfindungsgemäße Verbindungen | % Wirkung mit 500 | 250 | 100 ppm |
|---|---|---|---|
| 2,2-Di-n-propyl-5-(4-fluorphenyl)-4-(imidazol-1-yl)-3-oxo-pentannitril | 100 | | 100 |
| 2,2-Di-n-propyl-5-(4-fluorphenyl)-3-hydroxy-4-(imidazol-1-yl)-pentannitril | 100 | | |
| 2,2-Dimethyl-3-hydroxy-4-(imidazol-1-yl)-5-(4-methylphenyl)-pentannitril | 100 | | |
| 2,2-Diäthyl-5-(4-fluorphenyl)-3-hydroxy-4-(1,2,4-triazol-1-yl)-pentannitril | 100 | | |
| 5-(4-Chlorphenyl)-2,2-diäthyl-3-oxo-4-(1,2,4-triazol-1-yl)-pentannitril | 100 | | 100 |
| 2,2-Dimethyl-5-(4-methylphenyl)-3-oxo-4-(1,2,4-triazol-1-yl)-pentannitril | 100 | | 100 |
| 5-(4-Chlorphenyl)-2,2-diäthyl-3-hydroxy-4-(1,2,4-triazol-1-yl)-pentannitril, Naphthalin-1,5-disulfonat | 100 | | 100 |
| 2,2-Dimethyl-3-hydroxy-5-(4-methylphenyl)-4-(1,2,4-triazol-1-yl)-pentannitril | 100 | | |
| 2,2-Diäthyl-5-(4-methylphenyl)-3-oxo-4-(1,2,4-triazol-1-yl)-pentannitril | 100 | | |
| 2,2-Dimethyl-4-(imidazol-1-yl)-5-(4-methylphenyl)-3-oxo-pentannitril | 100 | | |
| 5-(2-Chlorphenyl)-2,2-dimethyl-3-oxo-4-(1,2,4-triazol-1-yl)-pentannitril | 100 | | |
| 5-(2-Chlorphenyl)-2,2-dimethyl-3-hydroxy-4-(imidazol-1-yl)-pentannitril | >90 | | |
| 2,2-Diäthyl-5-(4-fluorphenyl)-3-hydroxy-4-(1,2,4-triazol-1-yl)-pentannitril, Naphthalin-1,5-disulfonat | 100 | | 100 |
| 2,2-Diäthyl-5-(4-fluorphenyl)-3-hydroxy-4-(1,2,4-triazol-1-yl)-pentannitril, Hydronitrat | 100 | | 100 |
| 2,2-Di-n-propyl-5-(4-fluorphenyl)-3-hydroxy-4-(1,2,4-triazol-1-yl)-pentannitril, Naphthalin-1,5-disulfonat | 100 | | 100 |

-57-

Postanschrift: Schering Aktiengesellschaft, Postfach 65 03 11, D-1000 Berlin 65 · Für Besucher: Berlin-Wedding, Müllerstraße 170-178 Telegramme Scheringchemie Be
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Ham
Sitz der Gesellschaft: Berlin und Bergkamen Handelsregister: AG Charlottenburg 93 HRB 283 und AG Kamen HRB 0061 Berliner Commerzbank AG, Berlin, Konto 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto 2415008, Bankleitzahl 100 700 00 Postscheckamt Berlin West, Konto-Nr. 1175-101 Bankleitzahl 100 100 10

| Erfindungsgemäße Verbindungen | % Wirkung mit 500 | 250 | 100 ppm |
|---|---|---|---|
| 2,2-Di-n-propyl-5-(4-fluorphenyl)-3-hydroxy-4-(1,2,4-triazol-1-yl)-pentannitril, Hydronitrat | | 100 | 100 |
| 2,2-Diäthyl-5-(4-fluorphenyl)-3-hydroxy-4-(imidazol-1-yl)-pentannitril | | 100 | |
| 2,2-Dimethyl-5-(2-methylphenyl)-3-oxo-4-(1,2,4-triazol-1-yl)-pentannitril | | 100 | |
| 2,2-Dimethyl-5-(2-methylphenyl)-3-hydroxy-4-(1,2,4-triazol-1-yl)-pentannitril | | 100 | 100 |
| 2,2-Diäthyl-5-(2,4-dichlorphenyl)-3-oxo-4-(1,2,4-triazol-1-yl)-pentannitril | | 100 | 100 |
| 2,2-Diäthyl-5-(2,4-dichlorphenyl)-3-oxo-4-(imidazol-1-yl)-pentannitril | | 100 | 100 |
| 2,2-Diäthyl-5-(2,4-dichlorphenyl)-3-hydroxy-4-(imidazol-1-yl)-pentannitril | | 100 | |
| 2,2-Diäthyl-5-(2,4-dichlorphenyl)-3-hydroxy-4-(1,2,4-triazol-1-yl)-pentannitril | | 100 | 100 |
| 5-(2-Chlorphenyl)-2,2-dimethyl-3-hydroxy-4-(1,2,4-triazol-1-yl)-pentannitril | | 100 | |
| 5-(2-Chlorphenyl)-2,2-dimethyl-3-oxo-4-(1,2,4-triazol-1-yl)-pentannitril, Hydronitrat | | 100 | 100 |
| 5-(2-Chlorphenyl)-2,2-dimethyl-3-oxo-4-(imidazol-1-yl)-pentannitril | | 100 | 100 |
| 5-(2-Chlorphenyl)-2,2-diäthyl-3-oxo-4-(1,2,4-triazol-1-yl)-pentannitril | | 100 | 100 |
| 5-(2-Chlorphenyl)-2,2-diäthyl-3-hydroxy-4-(1,2,4-triazol-1-yl)-pentannitril | | 100 | 100 |
| 5-(4-Chlorphenyl)-2,2-di-n-propyl-3-oxo-4-(1,2,4-triazol-1-yl)-pentannitril | | 100 | 100 |
| 5-(4-Chlorphenyl)-2,2-di-n-propyl-3-oxo-4-(1,2,4-triazol-1-yl)-pentannitril, Hydronitrat | | 100 | 100 |
| 5-(4-Chlorphenyl)-2,2-di-n-propyl-4-(imidazol-1-yl)-3-oxo-pentannitril | | 100 | 100 |

-58-

Postanschrift: Schering Aktiengesellschaft, Postfach 65 03 11, D-1000 Berlin 65 · Für Besucher: Berlin-Wedding, Müllerstraße 170-178 Telegramme: Scheringchemie B
Vorstand: Dr. Herbert Asmis, Dr. Christian Brunn, Dr. Heinz Hannse, Horst Kramp, Dr. Klaus Pohle, Dr. Horst Witzel Vorsitzender des Aufsichtsrats: Hans-Jürgen Har
Sitz der Gesellschaft: Berlin und Bergkamen Handelsregister: AG Charlottenburg 93 HRB 283 und AG Kamen HRB 0061 Berliner Commerzbank AG, Berlin, Kto:
108700600, Bankleitzahl 100 400 00 Berliner Handels- und Frankfurter Bank, Berlin, Konto-Nr. 70045224, Bankleitzahl 100 202 00 Deutsche Bank Berlin AG, Kto:

| Erfindungsgemäße Verbindungen | % Wirkung mit 500 | 250 | 100 ppm |
|---|---|---|---|
| 5-(2,4-Dichlorphenyl)-2,2-di-n-propyl-3-oxo-4-(1,2,4-triazol-1-yl)-pentannitril | 100 | | 100 |
| 5-(2,4-Dichlorphenyl)-2,2-di-n-propyl-3-hydroxy-4-(1,2,4-triazol-1-yl)-pentannitril | 100 | | 100 |
| 5-(4-Chlorphenyl)-2,2-di-n-propyl-3-hydroxy-4-(imidazol-1-yl)-pentannitril | | 95 | |
| 5-(4-Chlorphenyl)-2,2-di-n-propyl-3-hydroxy-4-(1,2,4-triazol-1-yl)-pentannitril | | 93 | |
| 3-Oxo-2,2-pentamethylen-5-phenyl-4-(1,2,4-triazol-1-yl)-pentannitril | | 90 | |
| 3-Hydroxy-2,2-pentanmethylen-5-phenyl-4-(1,2,4-triazol-1-yl)-pentannitril | | 98 | |
| 5-(4-Chlorphenyl)-3-oxo-2,2-pentamethylen-4-(1,2,4-triazol-1-yl)-pentannitril | | 80 | |
| 5-(2,4-Dichlorphenyl)-3-oxo-2,2-pentamethylen-4-(1,2,4-triazol-1-yl)-pentannitril | | 100 | |
| 5-(4-Chlorphenyl)-3-hydroxy-2,2-pentamethylen-4-(1,2,4-triazol-1-yl)-pentannitril | | 98 | |
| 5-(2,4-Dichlorphenyl)-3-hydroxy-2,2-pentamethylen-4-(1,2,4-triazol-1-yl)-pentannitril | | 85 | |
| 5-(2,4-Dichlorphenyl)-2,2-di-n-butyl-3-hydroxy-4-(1,2,4-triazol-1-yl)-pentannitril | | 100 | |

-59-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 102

Wirkung prophylaktischer Spritzbehandlung gegen Kürbismehltau(Erysiphe cichoracearum)

Junge Kürbispflanzen mit mindestens 2 gut entwickelten Laubblättern wurden mit angegebener Konzentration tropfnaß gespritzt. Nach Antrocknen des Spritzbelages wurden die behandelten Pflanzen und unbehandelte Kontrollpflanzen durch Aufstäuben trockener Mehltausporen von Erysiphe cichoracearum inokuliert. Die Versuchspflanzen wurden im Gewächshaus bei 24°C inkubiert. Nach einer Woche wurde der prozentuale Anteil befallener Blattfläche notiert.
Wie die Tabelle zeigt, wurden ausgezeichnete und zum Teil noch bei der Konzentration von nur 10 ppm systemische Schutzwirkungen mit den erfindungsgemäßen Substanzen erzielt.

(s bedeutet in der Tabelle das Vorliegen systemischer Wirkung, erkennbar daran, daß auch der Zuwachs der Kürbispflanzen von Mehltau frei blieb)

| Erfindungsgemäße Verbindungen | % Wirkung mit 250 ppm | 10 ppm |
|---|---|---|
| 2,2-Dimethyl-3-oxo-5-phenyl-4-(1,2,4-triazol-1-yl)-pentannitril | 100s | 100 |
| 2,2-Dimethyl-3-hydroxy-5-phenyl-4-(1,2,4-triazol-1-yl)-pentannitril | 100s | 100 |
| 2,2-Dimethyl-4-(imidazol-1-yl)-3-oxo-5-phenyl-pentannitril | 100s | 100 |
| 2,2-Dimethyl-3-hydroxy-4-(imidazol-1-yl)-5-phenylpentannitril | 100 | 100 |
| 5-(4-Chlorphenyl)-2,2-dimethyl-3-oxo-4-(1,2,4-triazol-1-yl)-pentannitril | 100 | 100 |
| 5-(4-Chlorphenyl)-2,2-dimethyl-3-hydroxy-4-(1,2,4-triazol-1-yl)-pentannitril | 100 | 100 |
| 5-(4-Chlorphenyl)-2,2-dimethyl-4-(imidazol-1-yl)-3-oxo-pentannitril | 100 | 100 |

-60-

| Erfindungsgemäße Verbindungen | % Wirkung mit 250 ppm | 10 ppm |
|---|---|---|
| 5-(4-Chlorphenyl)-2,2-dimethyl-3-hydroxy-4-(imidazol-1-yl)-pentannitril | 100 | 100 |
| 5-(2,4-Dichlorphenyl)-2,2-dimethyl-3-oxo-4-(1,2,4-triazol-1-yl)-pentannitril | 100s | 100 |
| 5-(2,4-Dichlorphenyl)-2,2-dimethyl-3-hydroxy-4-(1,2,4-triazol-1-yl)-pentannitril | 100s | 100 |
| 5-(2,4-Dichlorphenyl)-2,2-dimethyl-3-hydroxy-4-(imidazol-1-yl)-pentannitril | 100 | 100 |
| 2,2-Diäthyl-3-oxo--5--phenyl-4-(1,2,4-triazol--1-yl)-pentannitril | 100s | 100s |
| 2,2-Di-äthyl-3-oxo-5--phenyl-4-(1,2,4-triazol--1-yl)-pentannitril | 100s | 100s |
| 2,2-Di-äthyl-3-hydroxy-5-phenyl-4-(1,2,4-triazol-1-yl)-pentannitril | 100s | 100s |
| 2,2-Di-n-propyl-3-oxo-5--phenyl-4-(1,2,4-triazol--1-yl)-pentannitril | 100s | 100s |
| 5-(4-Chlorphenyl)-2,2--dimethyl-3-oxo-4-(1,2,4--triazol-1-yl)-pentannitril, Hydronitrat | 100s | 100 |
| 2,2-Dimethyl-3-oxo-5-phenyl--4-(1,2,4-triazol-1-yl)--pentannitril, Hydronitrat | 100 | 100 |
| 5-(2,4-Dichlorphenyl)-2,2-dimethyl-3-oxo-4-(1,2,4-triazol--1-yl)-pentannitril, Hydronitrat | 100s | 100 |
| 5-(4-Chlorphenyl)-2,2-dimethyl-3-oxo-4-(1,2,4-triazol--1-yl)-pentannitril, Naphthalin-1,5-disulfonat | 100s | 95 |
| 5-(4-Chlorphenyl)-2,2-diäthyl-3-hydroxy-4-(1,2,4--triazol-1-yl)-pentannitril | 100s | 100s |

-61-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie :
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Ha
Sitz der Gesellschaft: Berlin und Bergkamen · Handelsregister: AG Charlottenburg 93 HRB 283 und AG Kamen HRB 0061 · Berliner Commerzbank AG, Berlin, Kon
108700600 Bankleitzahl 100 400 00 · Berliner Handels- und Frankfurter Bank, Berlin, Konto-Nr. 70045224 Bankleitzahl 100 202 00 · Deutsche Bank Berlin AG, Kon
2415008 Bankleitzahl 100 700 00 · Postscheckamt Berlin West, Konto-Nr. 1175-101, Bankleitzahl 100 100 10

| Erfindungsgemäße Verbindungen | % Wirkung mit 250 ppm | 10 ppm |
|---|---|---|
| 5-(4-Chlorphenyl)-2,2-diäthyl-3-oxo-4-(1,2,4-triazol-1-yl)-pentannitril, Naphthalin-1,5-disulfonat | 100s | 100 |
| 2,2-Diäthyl-3-hydroxy-5-phenyl-4-(imidazol-1-yl)-pentannitril | 100s | 95 |
| 2,2-Di-n-propyl-3-oxo-5-phenyl-4-(1,2,4-triazol-1-yl)-pentannitril, Naphthalin-1,5-disulfonat | 100 | 100 |
| 2,2-Di-n-propyl-3-hydroxy-5-phenyl-4-(1,2,4-triazol-1-yl)-pentannitril | 100s | 100s |
| 2,2-Di-n-propyl-3-oxo-5-phenyl-4-(imidazol-1-yl)-pentannitril | 100s | 100s |
| 2,2-Di-n-propyl-3-hydroxy-5-phenyl-4-(imidazol-1-yl)-pentannitril | 100 | 100 |
| 2,2,Di-n-propyl-3-hydroxy-5-phenyl-4-(imidazol-1-yl)-pentannitril, Hydronitrat | 100s | 100 |
| 2,2-Di-n-propyl-3-oxo-5-phenyl-4-(1,2,4-triazol-1-yl)-pentannitril, Hydronitrat | 100s | 100s |
| 2,2-Di-n-propyl-3-oxo-5-phenyl-4-(imidazol-1-yl)-pentannitril, Hydronitrat | 100s | 100s |
| 2,2-Diäthyl-3-oxo-5-phenyl-4-(imidazol-1-yl)-pentannitril, Hydronitrat | 100s | 100s |
| 2,2-Dimethyl-3-oxo-5-phenyl-4-(1,2,4-triazol-1-yl)-pentannitril, Naphthalin-1,5-disulfonat | 100s | 100 |
| 5-(2,4-Dichlorphenyl)-2,2-dimethyl-3-oxo-4-(1,2,4-triazol-1-yl)-pentannitril | 100s | 100s |
| 2,2-Diäthyl-3-oxo-5-phenyl-4-(1,2,4-triazol-1-yl)-pentannitril | 100s | 100s |
| 2,2-Diäthyl-3-oxo-5-phenyl-4-(1,2,4-triazol-1-yl)-pentannitril, Naphthalin-1,5-disulfonat | 100 | 100 |

-62-

| Erfindungsgemäße Verbindungen | % Wirkung mit 250 ppm | 10 ppm |
|---|---|---|
| 2,2-Dimethyl-5-(4-fluorphenyl)-3-oxo-4-(1,2,4-triazol-1-yl)-pentannitril | 100 | 95 |
| 2,2-Diäthyl-5-(4-fluorphenyl)-3-oxo-4-(1,2,4-triazol-1-yl)-pentannitril | 100s | 100 |
| 2,2-Di-n-propyl-5-(4-fluorphenyl)-3-oxo-4-(1,2,4-triazol-1-yl)-pentannitril | 100s | 100 |
| 2,2-Dimethyl-5-(4-fluorphenyl)-3-hydroxy-4-(1,2,4-triazol-1-yl)-pentannitril | 100s | 100s |
| 2,2-Dimethyl-5-(4-fluorphenyl)-3-oxo-4-(1,2,4-triazol-1-yl)-pentannitril, Hydronitrat | 100 | 95 |
| 2,2-Dimethyl-5-(4-fluorphenyl)-3-hydroxy-4-(1,2,4-triazol-1-yl)-pentannitril, Hydronitrat | 100s | 100 |
| 2,2-Diäthyl-5-(4-fluorphenyl)-3-oxo-4-(1,2,4-triazol-1-yl)-pentannitril, Hydronitrat | 100s | 95 |
| 2,2-Di-n-propyl-5-(4-fluorphenyl)-3-oxo-4-(1,2,4-triazol-1-yl)-pentannitril, Hydronitrat | 100s | 100 |
| 2,2-Dimethyl-5-(4-fluorphenyl)--3-oxo-4-(1,2,4-triazol-1-yl)-pentannitril, Naphthalin-1,5-disulfonat | 100 | 95 |
| 2,2-Dimethyl-5-(4-fluorphenyl)-3-hydroxy-4-(1,2,4-triazol-1-yl)-pentannitril, Naphthalin-1,5-disulfonat | 100s | 100 |
| 2,2-Diäthyl-5-(4-fluorphenyl)-3-oxo-4-(1,2,4-triazol-1-yl)-pentannitril, Naphthalin-1,5-disulfonat | 100s | 100 |
| 2,2-Diäthyl-5-(4-fluorphenyl)-3-hydroxy-4-(1,2,4-triazol-1-yl)-pentannitril | 100s | 100s |
| 2,2-Di-n-propyl-5-(4-fluorphenyl)-3-hydroxy-4-(1,2,4-triazol-1-yl)-pentannitril | 100s | 100s |

-63-

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG, Berlin, Konto-Nr. 108700600 Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008 Bankleitzahl 100 700 00 Postscheckamt Berlin West Konto-Nr. 1175-101, Bankleitzahl 100 100 10

| Erfindungsgemäße Verbindungen | % Wirkung mit 250 ppm | 10 ppm |
|---|---|---|
| 2,2-Di-n-butyl-5-(2,4-dichlorphenyl)-3-oxo-4-(1,2,4-triazol-1-yl)-pentannitril | 100 | 93 |
| 5-(2-Chlorphenyl)-2,2-di-n-propyl-3-oxo-4-(1,2,4-triazol-1-yl)-pentannitril | 100 s | 100 |
| 5-(2-Chlorphenyl)-2,2-di-n-propyl-3-hydroxy-4-(1,2,4-triazol-1-yl)-pentannitril | 100 s | 100 s |

-64-

**Postanschrift: Schering Aktiengesellschaft, Postfach 65 03 11, D-1000 Berlin 65** · Für Besucher: Berlin-Wedding, Müllerstraße 170-178 · Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 103

Wirkung prophylaktischer Spritzbehandlung gegen Gerstenzwergrost Puccinia hordei

Junge Gerstenpflanzen im Stadium des ersten Blattes wurden mit angegebener Konzentration tropfnaß gespritzt. Nach Antrocknen des Spritzbelages wurden die behandelten Pflanzen sowie unbehandelte Kontrollpflanzen durch Überstreichen mit Gersten-zwergrost befallenen Pflanzen inokuliert. In einer Pflanzenwuchskammer bei 15°C wurden die Pflanzen 10 Tage kultiviert, die beiden ersten Tage in nahezu feuchtgesättigter Luft.
Am Schluß wurde der prozentuale Anteil rostbefallener Blattfläche notiert.

| Erfindungsgemäße Verbindungen | % Wirkung mit 500 ppm | 100 ppm |
|---|---|---|
| 2,2-Dimethyl-3-oxo-5-phenyl-4-(1,2,4-triazol-1-yl)-pentannitril | 80 | - |
| 2,2-Dimethyl-4-(imidazol-1-yl)-3-oxo-5-phenyl-pentannitril | 80 | - |
| 5-(4-Chlorphenyl)-2,2-dimethyl-3-oxo-4-(1,2,4-triazol-1-yl)-pentannitril | 70 | - |
| 5-(2,4-Dichlorphenyl)-2,2-dimethyl-3-hydroxy-4-(imidazol-1-yl)-pentannitril | 90 | - |
| 2,2-Diäthyl-3-hydroxy-5-phenyl-4-(1,2,4-triazol-1-yl)-pentannitril | 95 | 100 |
| 2,2-Di-n-propyl-3-oxo-5-phenyl-4-(1,2,4-triazol-1-yl)-pentannitril | 95 | 90 |
| 2,2-Di-äthyl-3-hydroxy-5-phenyl-4-(1,2,4-triazol-1-yl)-pentannitril | 100 | 80 |
| 2,2-Di-n-propyl-3-oxo-5-phenyl-4-(1,2,4-triazol-1-yl)-pentannitril | 90 | 70 |

-65-

Postanschrift: Schering Aktiengesellschaft, Postfach 65 03 11, D-1000 Berlin 65 · Für Besucher Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG Konto-Nr. 2415008, Bankleitzahl 100 700 00 · Postscheckamt Berlin West Konto-Nr. 1175-101, Bankleitzahl 100 100 10

| Erfindungsgemäße Verbindungen | % Wirkung mit 500 ppm | 100 ppm |
|---|---|---|
| 5-(4-Chlorphenyl)-2,2-dimethyl-3-oxo-4-(1,2,4-triazol-1-yl)-pentannitril, Hydronitrat | 100 | - |
| 5-(2,4-Dichlorphenyl)-2,2-dimethyl-3-oxo-4-(1,2,4-triazol-1-yl)-pentannitril, Hydronitrat | 85 | - |
| 5-(4-Chlorphenyl)-2,2-dimethyl-3-oxo-4-(1,2,4-triazol-1-yl)-pentannitril, Naphthalin-1,5-disulfonat | 90 | - |
| 5-(4-Chlorphenyl)-2,2-diäthyl-3-hydroxy-4-(1,2,4-triazol-1-yl)-pentannitril | 100 | 95 |
| 5-(4-Chlorphenyl)-2,2-diäthyl-3-oxo-4-(1,2,4-triazol-1-yl)-pentannitril, Naphthalin-1,5-disulfonat | 100 | 100 |
| 2,2-Diäthyl-3-hydroxy-5-phenyl-4-(imidazol-1-yl)-pentannitril | 85 | - |
| 2,2-Di-n-propyl-3-oxo-5-phenyl-4-(1,2,4-triazol-1-yl)-pentannitril, Naphthalin-1,5-disulfonat | 95 | - |
| 2,2-Di-n-propyl-3-hydroxy-5-phenyl-4-(1,2,4-triazol-1-yl)-pentannitril | 100 | 80 |
| 2,2-Di-n-propyl-3-hydroxy-5-phenyl-4-(imidazol-1-yl)-pentannitril | 100 | - |
| 2,2,Di-n-propyl-3-hydroxy-5-phenyl-4-(imidazol-1-yl)-pentannitril, Hydronitrat | 100 | - |
| 2,2-Di-n-propyl-3-oxo-5-phenyl-4-(1,2,4-triazol-1-yl)-pentannitril, Hydronitrat | 100 | 70 |
| 2,2-Di-n-propyl-3-oxo-5-phenyl-4-(imidazol-1-yl)-pentannitril, Hydronitrat | 100 | - |



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG, Berlin, Konto-Nr. 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

| Erfindungsgemäße Verbindungen | % Wirkung mit 500 ppm | 100 ppm |
|---|---|---|
| 2,2-Diäthyl-3-oxo-5-phenyl-4-(imidazol-1-yl)-pentannitril, Hydronitrat | 100 | - |
| 2,2-Dimethyl-3-oxo-5-phenyl-4-(1,2,4-triazol-1-yl)-pentannitril, Naphthalin-1,5-disulfonat | 100 | - |
| 5-(2,4-Dichlorphenyl)-2,2-dimethyl-3-oxo-4-(1,2,4-triazol-1-yl)-pentannitril | 100 | - |
| 2,2-Diäthyl-3-oxo-5-phenyl-4-(1,2,4-triazol-1-yl)-pentannitril | 100 | - |
| 2,2-Diäthyl-3-oxo-5-phenyl-4-(1,2,4-triazol-1-yl)-pentannitril, Naphthalin-1,5-disulfonat | 100 | - |
| 2,2-Dimethyl-5-(4-fluorphenyl)-3-oxo-4-(1,2,4-triazol-1-yl)-pentannitril | 85 | - |
| 2,2-Diäthyl-5-(4-fluorphenyl)-3-oxo-4-(1,2,4-triazol-1-yl)-pentannitril | 100 | - |
| 2,2-Di-n-propyl-5-(4-fluorphenyl)-3-oxo-4-(1,2,4-triazol-1-yl)-pentannitril | 100 | - |
| 2,2-Dimethyl-5-(4-fluorphenyl)-3-hydroxy-4-(1,2,4-triazol-1-yl)-pentannitril | 95 | - |
| 2,2-Dimethyl-5-(4-fluorphenyl)-3-oxo-4-(1,2,4-triazol-1-yl)-pentannitril, Hydronitrat | 90 | - |
| 2,2-Dimethyl-5-(4-fluorphenyl)-3-hydroxy-4-(1,2,4-triazol-1-yl)-pentannitril, Hydronitrat | 95 | - |
| 2,2-Diäthyl-5-(4-fluorphenyl)-3-oxo-4-(1,2,4-triazol-1-yl)-pentannitril, Hydronitrat | 100 | - |
| 2,2-Di-n-propyl-5-(4-fluorphenyl)-3-oxo-4-(1,2,4-triazol-1-yl)-pentannitril, Hydronitrat | 100 | - |

-67-

| Erfindungsgemäße Verbindungen | % Wirkstoff mit 500 ppm | 100 ppm |
|---|---|---|
| 2,2-Dimethyl-5-(4-fluorphenyl)--3-oxo-4-(1,2,4--triazol-1-yl)-pentannitril, Naphthalin-1,5-disulfonat | > 90 | - |
| 2,2-Dimethyl-5-(4-fluorphenyl)-3-hydroxy-4-(1,2,4--triazol-1-yl)-pentannitril, Naphthalin-1,5-disulfonat | > 95 | - |
| 2,2-Diäthyl-5-(4-fluorphenyl)-3-oxo-4-(1,2,4--triazol-1-yl)-pentannitril, Naphthalin-1,5-disulfonat | 100 | - |
| 2,2-Diäthyl-5-(4-fluorphenyl)-3-hydroxy-4-(1,2,4-triazol--1-yl)-pentannitril | 98 | - |
| 2,2-Di-n-propyl-5-(4-fluorphenyl)-3-hydroxy-4-(1,2,4--triazol-1-yl)-pentannitril | 90 | - |
| 2,2-Di-n-propyl-5-(4-fluorphenyl)-4-(imidazol-1-yl)-3-oxo-pentannitril | 100 | |
| 5-(4-Chlorphenyl)-2,2-diäthyl-3-oxo-4-(1,2,4-triazol-1-yl)-pentannitril | 100 | |
| 2,2-Dimethyl-5-(4-methylphenyl)-3-oxo-4-(1,2,4-triazol-1-yl)-pentannitril | 100 | |
| 5-(4-Chlorphenyl)-2,2-diäthyl-3-hydroxy-4-(1,2,4-triazol-1-yl)-pentannitril, Naphthalin-1,5-disulfonat | 100 | |
| 2,2-Diäthyl-5-(4-fluorphenyl)-3-hydroxy-4-(1,2,4-triazol-1-yl)-pentannitril, Naphthalin-1,5-disulfonat | > 90 | |
| 2,2-Diäthyl-5-(4-fluorphenyl)-3-hydroxy-4-(1,2,4-triazol-1-yl)-pentannitril, Hydronitrat | > 90 | |
| 2,2-Dimethyl-5-(2-methylphenyl)-3-oxo-4-(1,2,4-triazol-1-yl)-pentannitril | > 90 | |
| 2,2-Dimethyl-5-(2-methylphenyl)-3-hydroxy-4-(1,2,4-triazol-1-yl)-pentannitril | 100 | |

-68-

| Erfindungsgemäße Verbindungen | % Wirkstoff 500 ppm | 100 ppm |
|---|---|---|
| 2,2-Diäthyl-5-(2,4-dichlorphenyl)-3-oxo-4-(1,2,4-triazol-1-yl)-pentannitril | > 90 | |
| 2,2-Diäthyl-5-(2,4-dichlorphenyl)-3-hydroxy-4-(1,2,4-triazol-1-yl)-pentannitril | 100 | 100 |
| 5-(2-Chlorphenyl)-2,2-dimethyl-3-hydroxy-4-(1,2,4-triazol-1-yl)-pentannitril | 100 | |
| 5-(2-Chlorphenyl)-2,2-diäthyl-3-oxo-4-(1,2,4-triazol-1-yl)-pentannitril | 100 | |
| 5-(2-Chlorphenyl)-2,2-diäthyl-3-hydroxy-4-(1,2,4-triazol-1-yl)-pentannitril | 100 | |
| 5-(4-Chlorphenyl)-2,2-di-n-propyl-3-oxo-4-(1,2,4-triazol-1-yl)-pentannitril | 100 | |
| 5-(4-Chlorphenyl)-2,2-di-n-propyl-3-oxo-4-(1,2,4-triazol-1-yl)-pentannitril, Hydronitrat | 100 | |
| 5-(4-Chlorphenyl)-2,2-di-n-propyl-4-(imidazol-1-yl)-3-oxo-pentannitril | 100 | |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen ·
Sitz der Gesellschaft: Berlin und Bergkamen · Handelsregister: AG Charlottenburg 93 HRB 283 und AG Kamen HRB 0061 · Berliner Commerzbank AG Berlin K. 108700600 Bankleitzahl 100 400 00 · Berliner Handels- und Frankfurter Bank Berlin Konto-Nr. 70045224 Bankleitzahl 100 202 00 · Deutsche Bank Berlin AG K. 2415008 Bankleitzahl 100 700 00 · Postscheckamt Berlin West, Konto-Nr. 1175-101, Bankleitzahl 100 100 10

## BEISPIEL 104

Wirkung prophylaktischer Spritzbehandlung gegen Fusarium culmorum an Kolbenhirse (Setaria italica)

Junge Hirsepflanzen von 3 bis 4 cm Höhe wurden mit angegebener Konzentration tropfnaß gespritzt. Nach Antrocknen des Spritzbelages wurden die behandelten Pflanzen sowie unbehandelte Kontrollpflanzen mit einer 30 % Biomalz enthaltenden wäßrigen Suspension der Fusariumsporen (800 000 pro Milliliter) besprüht und feucht im Gewächshaus bei 20 bis 22°C inkubiert. Nach 6 Tagen wurde der prozentuale Anteil befallener Blattfläche notiert.
Die erfindungsgemäße Verbindung ist dem Vergleichsmittel deutlich überlegen.

| Erfindungemäße Verbindung | % Wirkung mit 500 ppm |
|---|---|
| 2,2-Diäthyl-3-oxo-5-phenyl-4-(imidazol-1-yl)-pentannitril, Hydronitrat | 70 |
| Vergleichsmittel gemäß DE-OS 2 734 442 | |
| 1-(2,4-Dichlorphenyl)-4,4-dimethyl-2-(1,2,4-triazol-1-yl)-3-pentanol | 15 |



Postanschrift: Schering Aktiengesellschaft, Postfach 65 03 11, D-1000 Berlin 65 · Fur Besucher: Berlin-Wedding, Mullerstraße 170-178 Telegramme: Scheringchemie Be
Vorstand: Dr. Herbert Asmis Dr. Christian Bruhn Dr. Heinz Hannse, Horst Kramp, Dr. Klaus Pohle, Dr. Horst Witzel Vorsitzender des Aufsichtsrats: Hans-Jurgen Hama
Sitz der Gesellschaft Berlin und Bergkamen Handelsregister AG Charlottenburg 93 HRB 283 und AG Kamen HRB 0061 Berliner Commerzbank AG, Berlin Konto 108700600 Bankleitzahl 100 400 00 Berliner Handels- und Frankfurter Bank, Berlin, Konto-Nr 70045224, Bankleitzahl 100 202 00 Deutsche Bank Berlin AG, Konto 2415008, Bankleitzahl 100 700 00 Postscheckamt Berlin West, Konto-Nr 1175-101, Bankleitzahl 100 100 10

BEISPIEL 105

Wirkung prophylaktischer Spritzbehandlung gegen Pseudomonas phaseolicola an Bohnen

Wäßrige Wirkstoffzubereitungen wurden mit sterilisiertem, flüssigem Agarnährboden (2% Biomalz, 2% Agar) bei etwa 45°C in der Weise vermischt, daß die Mischung 250ppm Aktivsubstanz enthielt Die Mischung wurde in Polystyrol-Petrischalen von 8,5 cm Durchmesser 5 mm hoch gegossen. Nach dem Erkalten des Nährbodens wurde in die Mitte der Petrischale eine Suspension von Pseudomonas phaseolicola in destilliertem Wasser mittels 5 mm großer Impf-Öse abgetupft. Je Behandlung wurden 2 Schalen und von Unbehandelt 4 Schalen beimpft.
Nach 4 1/2 Wochen Inkubation bei 20 - 22°C im Dunkel wurde der Koloniedurchmesser bestimmt und nach Abzug der Inokulatgröße wie folgt zur Berechnung der bakteriziden Wirkung herangezogen:

$$100 - \frac{\text{Durchmesser ohne Inokulat in Behandelt} \cdot 100}{\text{Durchmesser ohne Inokulat in Unbehandelt}} = \%\ \text{Wirkung}$$

| Erfindungsgemäße Verbindungen | % Wirkung in 250 ppm |
|---|---|
| 5-(4-Chlorphenyl)-2,2-dimethyl-4-(imidazol-1-yl)-3-oxo-pentannitril | 100 |
| 2,2-Diäthyl-5-(4-fluorphenyl)-3-hydroxy-4-(1,2,4-triazol-1-yl)-pentannitril | 100 |
| 5-(2,4-Dichlorphenyl)-2,2-dimethyl-3-oxo-4-(1,2,4-triazol-1-yl)-pentannitril | 100 |
| 2,2-Diäthyl-3-oxo-5-phenyl-4-(1,2,4-triazol-1-yl)-pentannitril | 100 |
| 2,2-Diäthyl-3-oxo-5-phenyl-4-(imidazol-1-yl)-pentannitril | 100 |
| 2,2-Diäthyl-3-hydroxy-5-phenyl-4-(1,2,4-triazol-1-yl)-pentannitril | 100 |
| 2,2-Di-n-propyl-3-oxo-5-phenyl-4-(1,2,4-triazol-1-yl)-pentannitril | 100 |

-71-

Postanschrift: Schering Aktiengesellschaft, Postfach 65 03 11, D-1000 Berlin 65 · Für Besucher: Berlin-Wedding, Müllerstraße 170-178 Telegramme Scheringchemie B
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Har
Sitz der Gesellschaft: Berlin und Bergkamen Handelsregister: AG Charlottenburg 93 HRB 283 und AG Kamen HRB 0051 Berliner Commerzbank AG Berlin Konto 108700600, Bankleitzahl 100 400 00 Berliner Handels- und Frankfurter Bank, Berlin Konto-Nr. 70045224 Bankleitzahl 100 202 00 Deutsche Bank Berlin AG, Konto 2415008 Bankleitzahl 100 700 00 Postscheckamt Berlin West Konto-Nr. 1175-101, Bankleitzahl 100 100 10

| Erfindungsgemäße Verbindungen | % Wirkung in 250 ppm |
|---|---|
| 5-(4-Chlorphenyl)-2,2-dimethyl-3-oxo-4-(1,2,4-triazol-1-yl)-pentannitril, Hydronitrat | 100 |
| 2,2-Dimethyl-3-oxo-5-phenyl-4-(1,2,4-triazol-1-yl)-pentannitril, Hydronitrat | 100 |
| 2,2-Di-n-propyl-3-hydroxy-5-phenyl-4-(1,2,4-triazol-1-yl)-pentannitril | 100 |
| 2,2-Di-n-propyl-3-oxo-5-phenyl-4-(imidazol-1-yl)-pentannitril | 100 |
| 2,2-Di-n-propyl-3-oxo-5-phenyl-4-(imidazol-1-yl)-pentannitril, Hydronitrat | 100 |
| 2,2-Di-n-propyl-5-(4-fluorphenyl)-3-oxo-4-(1,2,4-triazol-1-yl)-pentannitril, Hydronitrat | 100 |

-72-

Die in den Ausführungsbeispielen 106 bis 109 beschriebenen Versuche wurden unter Gewächshausbedingungen durchgeführt. Im Vorauflaufverfahren wurden die Pflanzen im Primärblattstadium mit jeweils 0,5 kg Wirkstoff/ha als wäßrige Emulsionen oder Suspensionen behandelt. Die Auswertung erfolgte durch Bonitur (nach 11 Tagen im Beispiel 100; nach 6 Tagen im Beispiel 101; nach 11 Tagen im Versuch 102; nach 13 Tagen im Versuch 103).

Dabei wurde die durchschnittliche Länge der Pflanzen ermittelt und die prozentuale Wuchshemmung im Vergleich mit der Kontrolle (unbehandelt) berechnet.

Im Beispiel 100 wurden die Pflanzen nach der ersten Bonitur (a) drei Tage lang nicht gegossen und dann der Welkegrad nach dem Schema 0 bis 10 bonitiert (b), worin

0 = Pflanzen voll turgeszent
1 = Pflanze mit 10 % Trockenheitsschäden
2 = Pflanze mit 20 % Trockenheitsschäden
3 = Pflanze mit 30 % Trockenheitsschäden
4 = Pflanze mit 40 % Trockenheitsschäden
5 = Pflanze mit 50 % Trockenheitsschäden
6 = Pflanze mit 60 % Trockenheitsschäden
7 = Pflanze mit 70 % Trockenheitsschäden
8 = Pflanze mit 80 % Trockenheitsschäden
9 = Pflanze mit 90 % Trockenheitsschäden
10 = Pflanze total vertrocknet

bedeuten.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 106

a = Wuchshemmung in % - b = Welkegrad

| Erfindungsgemäße Verbindungen | Zuckerrübe | | R e i s | | Baumwolle | | S o j a | |
|---|---|---|---|---|---|---|---|---|
| | a | b | a | b | a | b | a | b |
| 2,2-Dimethyl-3-oxo-5-phenyl-4-(1,2,4-triazol-1-yl)-pentannitril | 57 | 0 | 8 | 2 | 14 | 2 | 77 | 0 |
| 2,2-Dimethyl-3-hydroxy-5-phenyl-4-(1,2,4-triazol-1-yl)-pentannitril | 29 | 0 | 15 | 2 | 36 | 0 | 77 | 0 |
| 2,2-Dimethyl-4-(imidazol-1-yl)-3-oxo-5-phenyl-pentannitril | 14 | 0 | 8 | 6 | 0 | 8 | 19 | 10 |
| 2,2-Dimethyl-3-hydroxy-4-(imidazol-1-yl)-5-phenylpentannitril | 0 | 0 | 15 | 4 | 7 | 8 | 46 | 8 |
| 5-(4-Chlorphenyl)-2,2-dimethyl-3-oxo-4-(1,2,4-triazol-1-yl)-pentannitril | 43 | 0 | 23 | 4 | 7 | 8 | 85 | 4 |
| 5-(4-Chlorphenyl)-2,2-dimethyl-3-hydroxy-4-(1,2,4-triazol-1-yl)-pentannitril | 57 | 0 | 23 | 2 | 57 | 4 | 88 | 0 |
| 5-(4-Chlorphenyl)-2,2-dimethyl-4-(imidazol-1-yl)-3-oxo-pentannitril | 29 | 0 | 0 | 2 | 0 | 8 | 38 | 8 |
| 5-(4-Chlorphenyl)-2,2-dimethyl-3-hydroxy-4-(imidazol-1-yl)-pentannitril | 29 | 0 | 0 | 4 | 0 | 8 | 31 | 8 |

-74

BEISPIEL 107

| Erfindungsgemäße Verbindungen | Wuchshemmung in % Zuckerrübe | Baumwolle | Reis | Sojabohne |
|---|---|---|---|---|
| 2,2-Diäthyl-3-oxo-5-phenyl-4-(1,2,4-triazol-1-yl)-pentannitril | 50 | 12 | 43 | 25 |
| 2,2-Diäthyl-3-oxo-5-phenyl-4-(imidazol-1-yl)-pentannitril | 0 | 0 | 57 | 12 |
| 2,2-Diäthyl-3-hydroxy-5-phenyl-4-(1,2,4-triazol-1-yl)-pentannitril | 50 | 0 | 43 | 47 |
| 2,2-Di-n-propyl-3-oxo-5-phenyl-4-(1,2,4-triazol-1-yl)-pentannitril | 25 | 0 | 43 | 0 |
| 5-(4-Chlorphenyl)-2,2-dimethyl-3-oxo-4-(1,2,4-triazol-1-yl)-pentannitril, Hydronitrat | 50 | 0 | 43 | 81 |
| 2,2-Dimethyl-3-oxo-5-phenyl-4-(1,2,4-triazol-1-yl)-pentannitril, Hydronitrat | 50 | 0 | 43 | 87 |
| 5-(2,4-Dichlorphenyl)-2,2-dimethyl-3-oxo-4-(1,2,4-triazol-1-yl)-pentannitril, Hydronitrat | 25 | 0 | 57 | 75 |

-75

**Postanschrift: Schering Aktiengesellschaft, Postfach 65 03 11, D-1000 Berlin 65** · Für Besucher: Berlin-Wedding, Müllerstraße 170-178 · Telegramme: Scheringchemie Berlin
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Erfindungsgemäße Verbindungen | Zuckerrübe | | Reis | | Baumwolle | | Soja | |
|---|---|---|---|---|---|---|---|---|
| | a | b | a | b | a | b | a | b |
| 5-(2,4-Dichlorphenyl)-2,2-dimethyl-3-oxo-4-(1,2,4-triazol-1-yl)-pentannitril | 29 | 0 | 0 | 2 | 0 | 8 | 62 | 4 |
| 5-(2,4-Dichlorphenyl)-2,2-dimethyl-3-hydroxy-4-(1,2,4-triazol-1-yl)-pentannitril | 29 | 0 | 0 | 2 | 21 | 2 | 73 | 0 |
| 5-(2,4-Dichlorphenyl)-2,2-dimethyl-3-hydroxy-4-(imidazol-1-yl)-pentannitril | 43 | 0 | 0 | 2 | 14 | 2 | 23 | 6 |
| Kontrolle | 0 | 0 | 0 | 4 | 0 | 8 | 0 | 10 |



Postanschrift: Schering Aktiengesellschaft, Postfach 65 03 11, D-1000 Berlin 65 · Für Besucher: Berlin-Wedding, Müllerstraße 170-178 Telegramme Scheringchemie B
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Har Sitz der Gesellschaft: Berlin und Bergkamen Handelsregister: AG Charlottenburg 93 HRB 283 und AG Kamen HRB 0061 Berliner Commerzbank AG, Berlin Konto 108700600, Bankleitzahl 100 400 00 Berliner Handels- und Frankfurter Bank, Berlin, Konto-Nr. 70045224, Bankleitzahl 100 202 00 Deutsche Bank Berlin AG Konto 2415008, Bankleitzahl 100 700 00 Postscheckamt Berlin West, Konto-Nr. 1175-101, Bankleitzahl 100 100 10

| Erfindungsgemäße Verbindungen | W U C H S H E M M U N G I N % Zuckerrübe | Baumwolle | Reis | Sojabohne |
|---|---|---|---|---|
| 5-(4-Chlorphenyl)-2,2-dimethyl-3-oxo-4-(1,2,4-triazol-1-yl)-pentannitril, Naphthalin-1,5-disulfonat | 50 | 0 | 43 | 81 |
| 5-(4-Chlorphenyl)-2,2-diäthyl-3-hydroxy-4-(1,2,4-triazol-1-yl)-pentannitril | 25 | 0 | 43 | 50 |
| 5-(4-Chlorphenyl)-2,2-diäthyl-3-oxo-4-(1,2,4-triazol-1-yl)-pentannitril, Naphthalin-1,5-disulfonat | 50 | 0 | 29 | 25 |
| 2,2-Di-n-propyl-3-hydroxy-5-phenyl-4-(1,2,4-triazol-1-yl)-pentannitril | 25 | 0 | 29 | 0 |
| 2,2-Di-n-propyl-3-oxo-5-phenyl-4-(imidazol-1-yl)-pentannitril | 0 | 12 | 29 | 0 |
| 2,2-Di-n-propyl-3-hydroxy-5-phenyl-4-(imidazol-1-yl)-pentannitril, Hydronitrat | 0 | 0 | 29 | 0 |
| 2,2-Di-n-propyl-3-oxo-5-phenyl-4-(1,2,4-triazol-1-yl)-pentannitril, Hydronitrat | 50 | 0 | 0 | 0 |



Postanschrift: Schering Aktiengesellschaft, Postfach 65 03 11, D-1000 Berlin 65 · Für Besucher: Berlin-Wedding, Müllerstraße 170-178 Telegramme Scheringchemie
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen H...
Sitz der Gesellschaft: Berlin und Bergkamen Handelsregister: AG Charlottenburg 93 HRB 283 und AG Kamen HRB 0061 Berliner Commerzbank AG Berlin, Ko... 108700600, Bankleitzahl 100 400 00 Berliner Handels- und Frankfurter Bank Berlin, Konto-Nr. 70045224 Bankleitzahl 100 202 00 Deutsche Bank Berlin AG, Ko... 2415008, Bankleitzahl 100 700 00 Postscheckamt Berlin West, Konto-Nr. 1175-101 Bankleitzahl 100 100 10

| Erfindungsgemäße Verbindungen | WUCHSHEMMUNG IN % Zuckerrübe | Baumwolle | Reis | Sojabohne |
|---|---|---|---|---|
| 2,2-Di-n-propyl-3-oxo-5-phenyl-4-(imidazol-1-yl)-pentannitril, Hydronitrat | 50 | 0 | 14 | 0 |
| 2,2-Diäthyl-3-oxo-5-phenyl-4-(imidazol-1-yl)-pentannitril, Hydronitrat | 25 | 0 | 43 | 0 |
| 2,2-Dimethyl-3-oxo-5-phenyl-4-(1,2,4-triazol-1-yl)-pentannitril, Naphthalin-1,5-disulfonat | 50 | 25 | 29 | 81 |
| 5-(2,4-Dichlorphenyl)-2,2-dimethyl-3-oxo-4-(1,2,4-triazol-1-yl)-pentannitril, Naphthalin-1,5-disulfonat | 25 | 0 | 43 | 0 |
| 2,2-Diäthyl-3-oxo-5-phenyl-4-(1,2,4-triazol-1-yl)-pentannitril, Hydronitrat | 50 | 25 | 14 | 31 |
| 2,2-Diäthyl-3-oxo-5-phenyl-4-(1,2,4-triazol-1-yl)-pentannitril, Naphthalin-1,5-disulfonat | 75 | 12 | 29 | 19 |
| 2,2-Dimethyl-5-(4-fluorphenyl)-3-oxo-4-(1,2,4-triazol-1-yl)-pentannitril, Naphthalin-1,5-disulfonat | 25 | 37 | 14 | 87 |



**Postanschrift: Schering Aktiengesellschaft, Postfach 65 03 11, D-1000 Berlin 65** · Für Besucher: Berlin-Wedding, Müllerstraße 170-178 · Telegramme Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 108

| Erfindungsgemäße Verbindungen | WUCHSHEMMUNG IN % Zuckerrübe | Reis | Baumwolle | Soja | Weizen | Mais |
|---|---|---|---|---|---|---|
| 5-(2-Chlorphenyl)-2,2-dimethyl-3-oxo-4-(1,2,4-triazol-1-yl)-pentannitril | 43 | 17 | 13 | 76 | 10 | 29 |
| 5-(2-Chlorphenyl)-2,2-dimethyl-3-hydroxy-4-(imidazol-1-yl)-pentannitril | 14 | 0 | 20 | 85 | 0 | 29 |

-79-

BEISPIEL 109

| Erfindungsgemäße Verbindungen | Wuchshemmung in % Baumwolle | Zuckerrübe | Reis | Soja |
|---|---|---|---|---|
| 2,2-Dimethyl-5-(4-fluorphenyl)-3-oxo-4-(1,2,4-triazol-1-yl)-pentannitril | 50 | 67 | 0 | 86 |
| 2,2-Diäthyl-5-(4-fluorphenyl)-3-hydroxy-4-(1,2,4-triazol-1-yl)-pentannitril | 14 | 17 | 0 | 0 |
| 2,2-Di-n-propyl-5-(4-fluorphenyl)-3-hydroxy-4-(1,2,4-triazol-1-yl)-pentannitril | 14 | 0 | 0 | 0 |



Postanschrift: Schering Aktiengesellschaft, Postfach 65 03 11, D-1000 Berlin 65 · Für Besucher: Berlin-Wedding, Müllerstraße 170-178 Telegramme Scheringchemie Be
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamm
Sitz der Gesellschaft Berlin und Bergkamen Handelsregister AG Charlottenburg 93 HRB 283 und AG Kamen HRB 0061 Berliner Commerzbank AG Berlin Konto 108700600, Bankleitzahl 100 400 00 Berliner Handels- und Frankfurter Bank, Berlin, Konto-Nr 70045224, Bankleitzahl 100 202 00 Deutsche Bank Berlin AG, Konto 2415008, Bankleitzahl 100 700 00 Postscheckamt Berlin West, Konto-Nr 1175-101, Bankleitzahl 100 100 10

BEISPIEL 110

Auf Pinto-Bohnen wurden die Prüfsubstanzen in einem acetonhaltigen Lanolinöl gelöst appliziert. Die Applikation erfolgte, nachdem die zweiten Internodien eine Länge von 2 mm erreicht hatten. Es wurden 10, 50 und 100 µg Wirkstoff aufgetragen. Die Auswertung erfolgte 3 Tage nach der Applikation. Die Primärblätter der Pflanzen waren m. o. w. stark dunkelgrün gefärbt - je nach Substanz. Das sich entwickelnde zweite Internodium wurde zum Teil sehr stark im Wachstum gehemmt.

| Erfindungsgemäße Verbindungen | Hemmung des Internodiumwachstums in % |
|---|---|
| 2,2-Di-n-propyl-3-hydroxy-5-phenyl-4-(imidazol-1-yl)-pentannitril | 7 |
| 2,2-Dimethyl-5-(4-fluorphenyl)-3-oxo-4-(1,2,4-triazol-1-yl)-pentannitril | 48 |
| 2,2-Di-n-propyl-5-(4-fluorphenyl)-3-oxo-4-(1,2,4-triazol-1-yl)-pentannitril | 91 |
| 2,2-Dimethyl-5-(4-fluorphenyl)-3-hydroxy-4-(1,2,4-triazol-1-yl)-pentannitril | 85 |
| 2,2-Dimethyl-5-(4-fluorphenyl)-3-oxo-4-(1,2,4-triazol-1-yl)-pentannitril, Hydronitrat | 84 |
| 2,2-Dimethyl-5-(4-fluorphenyl)-3-hydroxy-4-(1,2,4-triazol-1-yl)-pentannitril, Hydronitrat | 88 |
| 2,2-Diäthyl-5-(4-fluorphenyl)-3-oxo-4-(1,2,4-triazol-1-yl)-pentannitril, Hydronitrat | 51 |
| 2,2-Dimethyl-5-(4-fluorphenyl)-3-oxo-4-(1,2,4-triazol-1-yl)-pentannitril, Naphthalin-1,5-disulfonat | 76 |
| 2,2-Diäthyl-5-(4-fluorphenyl)-3-oxo-4-(1,2,4-triazol-1-yl)-pentannitril, Naphthalin-1,5-disulfonat | 34 |

-81-

| Erfindungsgemäße Verbindungen | Hemmung des Internodiumwachstums in % |
|---|---|
| 2,2-Dimethyl-5-(4-methylphenyl)-3-oxo-4-(1,2,4-triazol-1-yl)-pentannitril | 36 |
| 5-(4-Chlorphenyl)-2,2-diäthyl-3-hydroxy-4-(1,2,4-triazol-1-yl)-pentannitril, Naphthalin-1,5-disulfonat | 33 |
| 2,2-Dimethyl-3-hydroxy-5-(4-methylphenyl)-4-(1,2,4-triazol-1-yl)-pentannitril | 85 |
| 2,2-Dimethyl-4-(imidazol)-5-(4-methylphenyl)-3-oxopentannitril | 63 |
| 2,2-Diäthyl-5-(4-methylphenyl)-3-oxo-4-(1,2,4-triazol-1-yl)-pentannitril | 13 |

-82-


Vorstand: Dr. Herbert Asmis · Dr. Christian Bruhn · Dr. Heinz Hannse · Horst Kramp · Dr. Klaus Pohle · Dr. Horst Witzel · Vorsitzender des Aufsichtsrats: Hans-Jürgen Ha… Sitz der Gesellschaft: Berlin und Bergkamen · Handelsregister: AG Charlottenburg 93 HRB 283 und AG Kamen HRB 0061 · Berliner Commerzbank AG Berlin Kon… 108700600 Bankleitzahl 100 400 00 · Berliner Handels- und Frankfurter Bank, Berlin Konto-Nr. 70045224 Bankleitzahl 100 202 00 · Deutsche Bank Berlin AG Kon… 2415008, Bankleitzahl 100 700 00 · Postscheckamt Berlin West Konto-Nr. 1175-101 Bankleitzahl 100 100 10

BEISPIEL 111

Im Gewächshaus wurden die aufgeführten Pflanzen vor dem Auflaufen und nach dem Auflaufen mit der erfindungsgemäßen Verbindung in den angegebenen Aufwandmengen behandelt.
Die erfindungsgemäßen Verbindungen wurden zu diesem Zweck als Suspension mit 500 Liter Wasser/ha gleichmäßig auf den Boden bzw. die Pflanzen ausgebracht. 3 Wochen nach der Behandlung wurde bonitiert nach dem Boniturschema 0 bis 4, wobei

4 = Vernichtung der Pflanzen

3 = vollkommene bis sehr starke Inhibition der Pflanzen

2 = starke bis mittlere Inhibition der Pflanzen

1 = geringe Inhibition der Pflanzen

0 = normales Wachstum der Pflanzen

bedeuten.
Aus der Tabelle geht hervor, daß Pflanzenarten durch die erfindungsgemäßen Verbindungen vollkommen in ihrer Entwicklung inhibiert werden, so daß sie als Konkurrenz ausgeschaltet werden können.
Dieser Effekt kann zur Unkrautbekämpfung benutzt werden.



Postanschrift: Schering Aktiengesellschaft, Postfach 65 03 11, D-1000 Berlin 65 · Für Besucher Berlin-Wedding, Müllerstraße 170-178 Telegramme Scheringchemie Be-
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamm
Sitz der Gesellschaft: Berlin und Bergkamen Handelsregister: AG Charlottenburg 93 HRB 283 und AG Kamen HRB 0061 Berliner Commerzbank AG, Berlin, Konto
108700600 Bankleitzahl 100 400 00 Berliner Handels- und Frankfurter Bank, Berlin, Konto-Nr. 70045224, Bankleitzahl 100 202 00 Deutsche Bank Berlin AG, Konto
2415008, Bankleitzahl 100 700 00 Postscheckamt Berlin West Konto-Nr. 1175-101 Bankleitzahl 100 100 10

| Erfindungsgemäße Verbindungen | Aufwandmenge Wirkstoff kg/ha | Sinapis VA | Sinapis NA | Solanum VA | Solanum NA | Lolium VA | Lolium NA | Setaria VA | Setaria NA | Chrysanthemum VA | Chrysanthemum NA | Beta vulgaris VA | Beta vulgaris NA | Gossypium VA | Gossypium NA |
|---|---|---|---|---|---|---|---|---|---|---|---|---|---|---|---|
| 5-(4-Chlorphenyl)-2,2-dimethyl-3-oxo-4-(1,2,4-triazol-1-yl)-pentannitril | 0,3 | 0 | 3 | 2 | 1 | 0 | 0 | 0 | 0 | 0 | 0 | 1 | 0 | 1 | 0 |
| | 3,0 | 2 | 4 | 3 | 3 | 2 | 1 | 2 | 1 | 2 | 0 | 3 | 2 | 3 | 1 |
| 5-(4-Chlorphenyl)-2,2-dimethyl-3-hydroxy-4-(1,2,4-triazol-1-yl)-pentannitril | 0,3 | 1 | 3 | 2 | 1 | 0 | 0 | 0 | 0 | 0 | 0 | 1 | 0 | 1 | 0 |
| | 3,0 | 3 | 4 | 3 | 3 | 2 | 0 | 1 | 0 | 3 | 0 | 3 | 1 | 3 | 2 |
| 5-(4-Chlorphenyl)-2,2-dimethyl-4-(imidazol-1-yl)-3-oxo-pentannitril | 0,3 | 0 | 2 | 2 | 2 | 0 | 0 | 0 | 0 | 0 | 0 | 1 | 0 | 0 | 0 |
| | 3,0 | 0 | 3 | 3 | 3 | 1 | 0 | 1 | 1 | 3 | 1 | 3 | 1 | 0 | 1 |
| 5-(4-Chlorphenyl)-2,2-dimethyl-3-hydroxy-4-(imidazol-1-yl)-pentannitril | 0,3 | 0 | 1 | 1 | 1 | 0 | 0 | 0 | 0 | 0 | 0 | 0 | 0 | 0 | 0 |
| | 3,0 | 0 | 2 | 2 | 3 | 0 | 0 | 0 | 0 | 0 | 0 | 0 | 0 | 0 | 0 |
| 2,2-Dimethyl-5-(4-fluorphenyl)-3-hydroxy-4-(1,2,4-triazol-1-yl)-pentannitril | 0,3 | 0 | 0 | 0 | 1 | 0 | 0 | 0 | 0 | 0 | 0 | 1 | 0 | 0 | 0 |
| | 3,0 | 0 | 1 | 2 | 3 | 1 | 1 | 1 | 0 | 3 | 0 | 3 | 1 | 3 | 0 |

-84-

BEISPIEL 112

Wirkung prophylaktischer Spritzbehandlung gegen Apfelschorf (Venturia inaequalis) im Freiland

Im Wachstum befindliche Apfeltriebe wurden mit 300 ppm Wirkstoff enthaltender Spritzflüssigkeit tropfnaß behandelt. Nach dem Antrocknen wurden diese Triebe sowie unbehandelte Kontrolltriebe mit einer Suspension von 345 000 Venturia - Konidien pro Milliliter 3%iger wässriger Glokoselösung besprüht unter Vermeidung des Abtropfens. Jeder Trieb wurde sofort mit einem Polyäthylenbeutel umhüllt, der nach 2 Tagen entfernt wurde. 12 Tage später wurde die Pflanzung zur Stimulierung etwaigen Befalls tagsüber alle halbe Stunde beregnet an zwei Tagen. Drei Wochen nach der Inokulation wurde der prozentuale Anteil der von sporulierender Venturia bedeckten Blattfläche geschätzt. Die Fungizidwirkung berechnete man wie folgt.

$$100 - \frac{\text{Befall in Behandelt} \cdot 100}{\text{Befall in Unbehandelt}} = \%\ \text{Wirkung}$$

Die erfindungsgemäßen Verbindungen lagen in 10%iger oder 20%iger wasserdispergierbaren Formulierungen vor.

Die Befunde zeigen die überlegene Wirkung der erfindungsgemäßen Verbindungen gegenüber dem Vergleichsmittel.

-85-

Postanschrift: Schering Aktiengesellschaft, Postfach 65 03 11, D-1000 Berlin 65 · Für Besucher: Berlin-Wedding, Müllerstraße 170-178 Telegramme Scheringchemie
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen H... Sitz der Gesellschaft: Berlin und Bergkamen Handelsregister: AG Charlottenburg 93 HRB 283 und AG Kamen HRB 0061 Berliner Commerzbank AG, Berlin Kto. 108700600 Bankleitzahl 100 400 00 Berliner Handels- und Frankfurter Bank, Berlin, Konto-Nr. 70045224 Bankleitzahl 100 202 00 Deutsche Bank Berlin AG, Kto. 2415008, Bankleitzahl 100 700 00 Postscheckamt Berlin West Konto-Nr. 1175-101 Bankleitzahl 100 100 10

| Erfindungsgemäße Verbindungen | % Wirkung mit 300 ppm |
|---|---|
| 2,2-Dimethyl-5-(2-methylphenyl)-3-oxo-4-(1,2,4-triazol-1-yl)-pentannitril | 99,5 |
| 2,2-Dimethyl-5-(2-methylphenyl)-3-hydroxy-4-(1,2,4-triazol-1-yl)-pentannitril | 96 |
| 2,2-Diäthyl-5-(2,4-dichlorphenyl)-3-oxo-4-(1,2,4-triazol-1-yl)-pentannitril | 100 |
| 2,2-Diäthyl-5-(2,4-dichlorphenyl)-3-oxo-4-(imidazol-1-yl)-pentannitril | 99 |
| 2,2-Diätyhl-5-(2,4-dichlorphenyl)-3-hydroxy-4-(imidazol-1-yl)-pentannitril | 95 |
| 2,2-Diäthyl-5-(2,4-dichlorphenyl)-3-hydroxy-4-(1,2,4-triazol-1-yl)-pentannitril | 100 |
| 5-(2-Chlorphenyl)-2,2-diäthyl-3-oxo-4-(1,2,4-triazol-1-yl)-pentannitril | 97 |
| 5-(4-Chlorphenyl)-2,2-di-n-propyl-3-oxo-4-(1,2,4-triazol-1-yl)-pentannitril, Hydronitrat | 95 |
| V e r g l e i c h s m i t t e l | |
| 3,3-Dimethyl-1-(4-phenyl-phenoxy)-1-(1,2,4-triazol-1-yl)-butan-2-ol | 94 |

-86-

Postanschrift: Schering Aktiengesellschaft, Postfach 65 03 11, D-1000 Berlin 65 · Für Besucher: Berlin-Wedding, Müllerstraße 170-178 · Telegramme Scheringchemie
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen ...
Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

Formular-Nr. 1439-4

# PATENTANSPRÜCHE

1. 4-Azolyl-pentannitrile der allgemeinen Formel

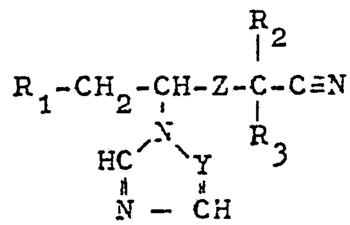

I

in der

$R_1$ einen gegebenenfalls ein- oder mehrfach, gleich oder verschieden durch Halogen, $(C_1-C_4)$-Alkyl, $(C_1-C_4)$-Alkoxy, $(C_1-C_4)$-Alkylthio, Trifluormethyl, Cyan und/oder die Nitrogruppe substituierten aromatischen Kohlenwasserstoffrest,

Y ein N-Atom oder die CH-Gruppe,

Z die Carbonyl- oder die CH(OH)-Gruppe und

$R_2$ und $R_3$ gleich oder verschieden sind und jeweils einen gegebenenfalls durch Halogen oder $(C_1-C_4)$-Alkoxy substituierten $(C_1-C_6)$-Alkylrest, einen $(C_3-C_6)$-Cycloalkylrest, einen $(C_3-C_6)$-Alkenyl- oder einen $(C_3-C_6)$-Alkinylrest, einen gegebenenfalls durch Halogen substituierten Phenyl-$(C_1-C_4)$-alkylrest oder gemeinsam einen Alkylenrest

$$-(CH_2)_n-$$

bedeuten, worin n die Zahlen 2, 3, 4, 5 und 6 darstellt und deren Säureadditionssalze mit anorganischen und organischen Säuren.

2. 4-Azolyl-pentannitrile gemäß Anspruch 1, worin $R_1$ Phenyl, Methylphenyl, Dimethylphenyl, Trimethylphenyl, Fluorphenyl, Difluorphenyl, Chlorphenyl, Dichlorphenyl, Trichlorphenyl, Bromphenyl, Dibromphenyl, Jodphenyl, Trifluormethylphenyl, Methoxyphenyl, Dimethoxyphenyl, Äthoxyphenyl, Methylthiophenyl, Äthylthiophenyl, Nitrophenyl,

-87-

Postanschrift: Schering Aktiengesellschaft, Postfach 65 03 11, D-1000 Berlin 65 · Für Besucher Berlin-Wedding, Müllerstraße 170-178 Telegramme Scheringchemie B

Vorstand: Dr. Herbert Asmis, Dr. Christian Bruhn, Dr. Heinz Hannse, Horst Krafft, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG Berlin Konto 108700600 Bankleitzahl 100 400 00 · Berliner Handels- und Frankfurter Bank Berlin Konto-Nr. 10045224 Bankleitzahl 100 202 00 · Deutsche Bank Berlin AG Konto 2415008 Bankleitzahl 100 700 00 · Postscheckamt Berlin West Konto-Nr. 1175-101 Bankleitzahl 100 100 10

Cyanphenyl, Naphthyl, Pyridyl, Furyl oder Thienyl, Y ein N-Atom oder die CH-Gruppe, Z die Carbonyl- oder CH(OH)-Gruppe und $R_2$ und $R_3$ gleich oder verschieden sind und jeweils Methyl, Äthyl, Propyl, n-Butyl, n-Pentyl, n-Hexyl, Isopropyl, Isobutyl, Cyclopropyl, Cyclopentyl, Cyclohexyl, 2-Propenyl, 2-Propinyl, Benzyl, Chlorbenzyl oder gemeinsam einen Alkylenrest

$$-(CH_2)_n-$$

bedeuten, worin n die Zahlen 2, 3, 4, 5 oder 6 darstellt.

3. 5-(4-Chlorphenyl)-2,2-dimethyl-4-(imidazol-1-yl)-3-oxo-pentannitril

4. 5-(4-Chlorphenyl-2,2-dimethyl-3-hydroxy-4-(imidazol-1-yl)-pentannitril

5. 5-(4-Chlorphenyl)-2,2-dimethyl-3-oxo-4-(1,2,4-triazol-1-yl)-pentannitril

6. 5-(4-Chlorphenyl)-2,2-dimethyl-3-hydroxy-4-(1,2,4-triazol-1-yl)-pentannitril

7. 2,2-Diäthyl-5-(4-Fluorphenyl)-3-hydroxy-4-(1,2,4-triazol-1-yl)-pentannitril

8. 5-(2,4-Dichlorphenyl)-2,2-dimethyl-3-oxo-4-(1,2,4-triazol-1-yl)-pentannitril

9. 2,2-Diäthyl-3-oxo-5-phenyl-4-(1,2,4-triazol-1-yl)-pentannitril

10. 2,2-Diäthyl-3-oxo-5-phenyl-4-(imidazol-1-yl)-pentannitril

11. 2,2-Diäthyl-3-hydroxy-5-phenyl-4-(1,2,4-triazol-1-yl)-pentannitril

12. 2,2-Di-n-propyl-3-oxo-5-phenyl-4-(1,2,4-triazol-1-yl)-pentannitril

-88-

13. 5-(4-Chlorphenyl)-2,2-dimethyl-3-oxo-4-(1,2,4-triazol-1-yl)-pentannitril, Hydronitrat
14. 2,2-Dimethyl-3-oxo-5-phenyl-4-(1,2,4-triazol-1-yl)-pentannitril, Hydronitrat
15. 2,2-Di-n-propyl-3-oxo-5-phenyl-4-(imidazol-1-yl)-pentannitril, Hydronitrat
16. 2,2-Diäthyl-3-oxo-5-phenyl-4-(imidazol-1-yl)-pentannitril, Hydronitrat
17. 2,2-Di-n-propyl-5-(4-fluorphenyl)-3-oxo-4-(1,2,4-triazol-1-yl)-pentannitril
18. 2,2-Dimethyl-5-(4-fluorphenyl)-3-hydroxy-4-(1,2,4-triazol-1-yl)-pentannitril
19. 5-(4-Chlorphenyl)-2,2-di-n-propyl-3-oxo-4-(1,2,4-triazol-1-yl)-pentannitril

-89-

20. Verfahren zur Herstellung von 4-Azolyl-Pentannitrilen gemäß Ansprüchen 1 bis 19, dadurch gekennzeichnet, daß man Verbindungen der allgemeinen Formel

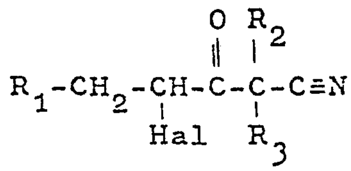
II

mit Verbindungen der allgemeinen Formel

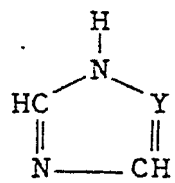
III

oder deren Metallsalzen in Gegenwart eines inerten Lösungsmittels und gegebenenfalls in Gegenwart eines Säureakzeptors unter Bildung von Verbindungen der allgemeinen Formel

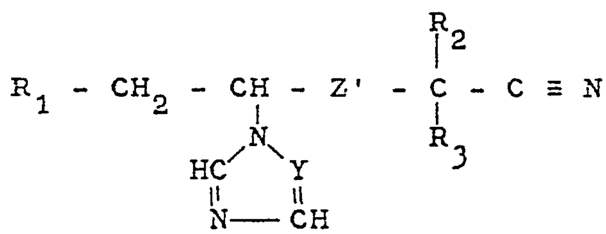
IV

umsetzt, die man gewünschtenfalls zu Verbindungen der allgemeinen Formel

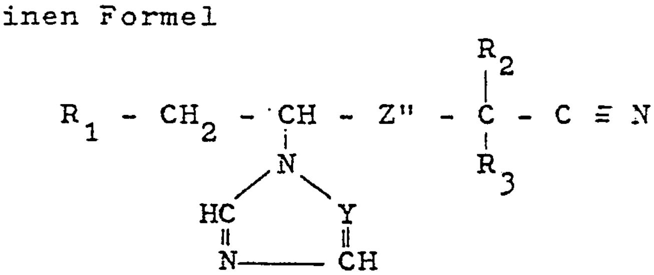
V

reduziert, worin $R_1$, $R_2$, $R_3$ und Y die oben genannte Bedeutung haben, Hal ein Halogenatom, vorzugsweise ein Bromatom,



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Z' die Carbonylgruppe und Z" die CH(OH)-Gruppe bedeuten.

21. Biozide Mittel, gekennzeichnet durch einen Gehalt an mindestens einer Verbindung gemäß den Ansprüchen 1 bis 19.

22. Biozide Mittel gemäß Anspruch 21 mit fungizider Wirkung.

23. Biozide Mittel gemäß Anspruch 21 mit pflanzenwachstumsregulatorischer Wirkung.

24. Biozide Mittel gemäß Anspruch 21 mit herbizider Wirkung.

25. Biozide Mittel gemäß Anspruch 21 mit bakterizider Wirkung.

-91-